# EUROPEAN PATENT SPECIFICATION

(11) **EP 2 129 773 B1**
(45) Date of publication and mention of the grant of the patent: **13.02.2013**
(21) Application number: 08799678.1
(22) Date of filing: 22.02.2008
(51) Int. Cl.: C12N 5/07, A61K 39/395, C07K 16/28

(54) **THERAPEUTIC APPLICATIONS OF ACTIVATION OF HUMAN ANTIGEN-PRESENTING CELLS THROUGH DECTIN-1**
THERAPEUTISCHE ANWENDUNGEN DER AKTIVIERUNG VON ZELLEN MIT MENSCHLICHEN ANTIGENEN DURCH DECTIN-1
APPLICATIONS THÉRAPEUTIQUES DES CELLULES PRÉSENTANT UN ANTIGÈNE HUMAIN PAR L'INTERMÉDIAIRE DE LA DECTINE-1

(30) Priority: 23.02.2007 US 891425 P
(43) Date of publication of application: 09.12.2009
(60) Divisional application: 12156717.6
(73) Proprietor: Baylor Research Institute, Dallas, TX 75204 (US)
(72) Inventor: BANCHEREAU, Jacques, F., Dallas, TX 75230 (US); OH, SangKon, Baltimore, MD 21237 (US); ZURAWSKI, Gerard, Midlothian, TX 76065 (US); ZURAWSKI, Sandra, Midlothian, TX 76065 (US); NI, Ling, Dallas, TX 75206 (US)
(74) Representative: Sonn & Partner Patentanwälte
(86) International application number: PCT/US2008/054798
(87) International publication number: WO 2008/118587

(56) References cited:
- US-B2- 7 129 039
- DATABASE BIOSIS [Online] BIOSCIENCES INFORMATION SERVICE, PHILADELPHIA, PA, US; November 2007 (2007-11), GRUENEBACH FRANK ET AL: "hDectin-1 mediates cross-presentation of epitopes via the cytosolic pathway" XP002563205 Database accession no. PREV200800217585
- "In vitro production of antigen-activated dendritic cell precursor for use as an immunogen in a vaccine; application in juvenile diabetes, multiple sclerosis, myasthenia gravis and atopic dermatitis therapy" VACCINE, BUTTERWORTH SCIENTIFIC. GUILDFORD, GB, vol. 12, no. 5, 1 January 1994 (1994-01-01), page 478, XP023711675 ISSN: 0264-410X [retrieved on 1994-01-01]
- PROUDFOOT OWEN ET AL: "Receptor-mediated delivery of antigens to dendritic cells: Anticancer applications" MOLECULAR PHARMACEUTICS, vol. 4, no. 1, January 2007 (2007-01), pages 58-72, XP002563197 ISSN: 1543-8384
- CARTER ROBERT W ET AL: "Preferential induction of CD4+ T cell responses through in vivo targeting of antigen to dendritic cell-associated C-type lectin-1" JOURNAL OF IMMUNOLOGY, AMERICAN ASSOCIATION OF IMMUNOLOGISTS, US, vol. 177, no. 4, 15 August 2006 (2006-08-15), pages 2276-2284, XP002503158 ISSN: 0022-1767
- "MANNOSE RECEPTOR-MEDIATED UPTAKE OF ANTIGENS STRONGLY ENHANCES HLA CLASS II-RESTRICTED ANTIGEN PRESENTATION BY CULTURED DENDRITIC CELL" EUROPEAN JOURNAL OF IMMUNOLOGY, WILEY - V C H VERLAG GMBH & CO. KGAA, DE, vol. 27, 1 September 1997 (1997-09-01), pages 2426-2435, XP000877415 ISSN: 0014-2980
- BROWN GORDON D: "Dectin-1: a signalling non-TLR pattern-recognition receptor" NATURE REVIEWS IMMUNOLOGY, vol. 6, no. 1, January 2006 (2006-01), pages 33-43, XP002563198 ISSN: 1474-1733
- RAMAKRISHNA ET AL.: 'Toll-like receptor activation enhances cell-mediated immunity induced by an antibody vaccine targeting human dendritic cells' JOURNAL OF TRANSLATION MEDICINE 2007 vol. 5, 25 January 2007, pages 1 - 14, XP008117462

## Description

The present invention relates in general to the field of antigen presentation and immune cell activation, and more particularly, to compositions and methods for the activation of immune cells through Dectin-1.

### BACKGROUND OF THE INVENTION

Without limiting the scope of the invention, its background is described in connection with dendritic cell activation.

Dendritic Cells play a pivotal role in controlling the interface of innate and acquired immunity by providing soluble and intercellular signals, followed by recognition of pathogens. These functions of DCs are largely dependent on the expression of specialized surface receptors, 'pattern recognition receptors' (PRRs), represented, most notably, by toll-like receptors (TLRs) and C-type lectins or lectin-like receptors (LLRs) (1-3).

In the current paradigm, a major role of TLRs is to alert DCs to produce interleukin 12 (IL-12) and other inflammatory cytokines for initiating immune responses. C-type LLRs operate as constituents of the powerful antigen capture and uptake mechanism of macrophages and DCs (1). Compared to TLRs, however, LLRs might have broader ranges of biological functions that include cell migration (4) and intercellular interactions (5). These multiple functions of LLRs might be due to the fact that LLRs, unlike TLRs, can recognize both self and non-self. However, the complexity of LLRs, including the redundancy of a number of LLRs expressed in immune cells, has been one of the major obstacles to understand the detailed functions of individual LLRs. In addition, natural ligands for most of these receptors remain unidentified. Nonetheless, evidence from recent studies suggests that LLRs, in collaboration with TLRs, may contribute to the activation of immune cells during microbial infections (6-14). Compared to other LLRs, Dectin-1 is known to have ITAM motif that delivers signals to activate cells expressing Dectin-1. However, most of the studies have been performed in the mouse model and the biological function of Dectin-1 expressed on human antigen presenting cells, including DCs, monocytes, and B cells, has not been well characterized. This is particularly important since there is not strict concordance between mouse and human for many of the LLRs.

### SUMMARY OF THE INVENTION

Herein disclosed are compositions and methods for making and using vaccines that specifically target (deliver) antigens to antigen-presenting cells for the purpose of eliciting potent and broad immune responses directed against the antigen. The purpose is primarily to evoke protective or therapeutic immune responses against the agent (pathogen or cancer) from which the antigen was derived. In addition the invention includes agents that are directly, or in concert with other agents, therapeutic through their specific engagement of a receptor called Dectin-1 that is expressed on antigen-presenting cells.

The present inventors show that Dectin-1 expression in human cells is restricted to antigen presenting cells, including DCs, Monocytes, and B cells. It is known that Dectin-1 expressed on mouse cells (macrophages) is downregulated by TLR4 ligand and IL-10, herein we show only TLR4 ligand can downregulate Dectin-1 expression on human DCs and IL-10 results in slightly increased expression of Dectin-1. More interestingly, Dectin-1 expressed on human DCs synergizes with TLR4 much more effectively than with TLR2, and the synergy between Dectin-1 and TLR4 results in dramatically increased production of cytokines and chemokines, particularly IL-12 that was not observed previously in mouse models. Human tonsil B cells can be divided into two groups: CD19⁺Dectin-1⁺ and CD19⁺Dectin-1⁻. These fmdings can also be used in therapeutic and preventive reagents based on anti-Dectin-1 agents.

Therefore, it was found herein that signaling through human Dectin-1 is a unique and functional, either alone or in collaboration with other cellular signals, in terms of cell (including DC) activation. Dectin-1-mediated cell activation is induced by particular anti-Dectin-1 mAbs, and therefore such anti-human Dectin-1 mAbs will be useful for developing reagents against diseases.

Herein disclosed are compositions and methods for increasing the effectiveness of antigen presentation by a Dectin-1-expressing antigen presenting cell by contacting the antigen presenting cell with an anti-Dectin-1-specific antibody or fragment thereof capable of activating the antigen presenting cell, wherein the antigen presenting cell is activated. Examples of antigen presenting cell include dendritic cells, peripheral blood mononuclear cells, monocytes, B cells, myeloid dendritic cells and combinations thereof. The antigen presenting cell may be cultured in vitro with GM-CSF and IL-4, interferon alpha, antigen and combinations thereof. Upon activation of the antigen presenting cells contacted with GM-CSF and IL-4 and a Dectin-1-specific antibody or fragment, an increase in the surface expression of CD86, CD80, and HLA-DR on the antigen presenting cell is obtained. When activating the antigen presenting cells with Interferon alpha and the Dectin-1-specific antibody or fragment thereof, the cells increase the surface expression of CD86, CD83, CD80, and HLA-DR.

Yet another method of the present disclosure includes contacting dendritic cell that has been contacted with GM-CSF and IL-4 or Interferon alpha to activate the dendritic cell, wherein the activated dendritic cells increases the surface expression of CD86, CD80, and HLA-DR. For dendritic cells that have been contacted with the anti-Dectin-1 antibody or fragments thereof and GM-CSF and IL-4 or Interferon alpha to activate the dendritic cell, the activated dendritic cells increases the secretion of IL-6, IL-8, IL-10, IL-12p40, IP-10, and MIP-1a and combinations thereof. Yet another method of activating dendritic cell includes contacting with GM-CSF and IL-4 or Interferon alpha and the Dectin-1-specific antibody or fragment thereof increases the activation in conjunction with signaling through CD40. It has been found that the dendritic cell contacted with GM-CSF and IL-4 or Interferon alpha and the Dectin-1-specific antibody or fragment thereof have increased co-stimulatory activity by dendritic cells of B cells and T cells.

Yet another method include contacting an antigen presenting cell with the anti-Dectin-1 antibody and co-activating the antigen presenting cell the activating through the TLR4 receptor, wherein the cells increase cytokine and chemokine production. Co-activating the antigen presenting cell by activating through the TLR4 receptor, wherein the cells increase secretion of IL-10, IL-1b, TNFα, IL-12p40 and combinations thereof.

Herein disclosed are methods further comprising the step of co-activating the antigen presenting cell the activating through the TLR4 receptor using at least one of a TLR4 ligand, an anti-TLR4 antibody of fragments thereof, an anti-TLR4-anti-Dectin-1 hybrid antibody or fragment thereof, an anti-TLR4-anti-Dectin-1 ligand conjugate. The Dectin-1-specific antibody or fragment may be one or more of the clones selected from 15E2.5, 11B6.4, 15F4.7, 14D6.3 and 9H7.6 and combinations thereof. The dendritic cells may be activated through the Dectin-1-receptor with the Dectin-1-specific antibody or fragment thereof to activate monocytes, dendritic cells, peripheral blood mononuclear cells, B cells and combinations thereof. The Dectin-1-specific antibody or fragment thereof may be bound to one half of a Cohesin/Dockerin pair. When the Dectin-1-specific antibody or fragment thereof is bound to one half of a Cohesin/Dockerin pair, the other half of the pair may be bound to one or more antigens, cytokines selected from interleukins, transforming growth factors (TGFs), fibroblast growth factors (FGFs), platelet derived growth factors (PDGFs), epidermal growth factors (EGFs), connective tissue activated peptides (CTAPs), osteogenic factors, and biologically active analogs, fragments, and derivatives of such growth factors, B/T-cell differentiation factors, B/T-cell growth factors, mitogenic cytokines, chemotactic cytokines and chemokines, colony stimulating factors, angiogenesis factors, IFN-α, IFN-β, IFN-γ, IL1, IL2, IL3, IL4, IL5, IL6, IL7, IL8, IL9, IL10, IL11, IL12, IL13, IL14, IL15, IL16, IL17, IL18, etc., leptin, myostatin, macrophage stimulating protein, platelet-derived growth factor, TNF-α, TNF-β, NGF, CD40L, CD137L/4-1BBL, human lymphotoxin-β, G-CSF, M-CSF, GM-CSF, PDGF, IL-1α, IL1- β, IP-10, PF4, GRO, 9E3, erythropoietin, endostatin, angiostatin, VEGF, transforming growth factor (TGF) supergene family include the beta transforming growth factors (for example TGF-β1, TGF-β2, TGF-β3); bone morphogenetic proteins (for example, BMP-1, BMP-2, BMP-3, BMP-4, BMP-5, BMP-6, BMP-7, BMP-8, BMP-9); heparin-binding growth factors (fibroblast growth factor (FGF), epidermal growth factor (EGF), platelet-derived growth factor (PDGF), insulin-like growth factor (IGF)); Inhibins (for example, Inhibin A, Inhibin B); growth differentiating factors (for example, GDF-1); and Activins (for example, Activin A, Activin B, Activin AB).

Herein disclosed are hybridoma and antibodies isolated therefrom that expressed a Dectin-1-specific antibody or fragment thereof, wherein the Dectin-1-specific antibody or fragment thereof activates an antigen presenting cell to express new surface markers, secrete one or more cytokines or both. The hybridoma may be selected from clone PAB1, PAB4, PAB5, PAB8, PAB10 and combinations thereof.

A method for enhancing B cell immune responses that includes triggering a Dectin-1 receptor on a dendritic cell with a Dectin-1 specific antibody or fragment thereof in the presence of antigen and an activator of TLR9, wherein a B cell that is contacted with the Dectin-1/TLR9 activated dendritic cell increases antibody production, secretes cytokines, increase B cell activation surface marker expression and combinations thereof. B cells activated using these dendritic cells secrete IL-8, MIP-1α, IL-6, TNFα and combinations thereof. Examples of B cells include plasma cells, which may also express Dectin-1 and/or increases production of IgM.

Herein disclosed are methods for enhancing B cell immune responses comprising triggering a Dectin-1 receptor on a B-cell with a Dectin-1 specific antibody or fragment thereof, wherein the B cell increases antibody production. B cells activated using the present invention increase production of secreted IL-8, MIP-1α, IL-6, TNFα and combinations thereof and/or increase production of IgG, IgM, IgA and combinations thereof.

Herein disclosed are methods for enhancing T cell activation by triggering a Dectin-1 receptor on a dendritic cell with a Dectin-1 specific antibody or fragment thereof and TLR4 receptor and contacting a T cell to the Dectin-1/TLR4 activated dendritic cell, wherein T cell activation is enhanced. The dendritic cells used for activation may be contacted with GM-CSF and IL-4, interferon alpha, antigen and combinations thereof. The Dectin-1 specific antibody or fragment thereof increases the secretion of IL-10, IL-15 and combinations thereof by the T cells. The T cells activated by the dendritic cells may increase the surface expression of 4-1BBL and/or proliferate.

Herein disclosed are anti-Dectin-1 immunoglobulins or portion thereof that is secreted from mammalian cells and an antigen bound to the immunoglobulin, wherein the immunoglobulin targets the antigen to antigen presenting cells. The antigen specific domain includes a full length antibody, an antibody variable region domain, an Fab fragment, a Fab' fragment, an F(ab)₂ fragment, and Fv fragment, and Fabc fragment and/or a Fab fragment with portions of the Fc domain.

Herein disclosed are vaccines that includes a dendritic cell activated with a Dectin-1-specific antibody or fragment thereof. The present invention also includes B cells, T cells or other immune cells that are activated either directly by the Dectin-1-specific antibody or fragments thereof and/or by antigen presenting cells, such as dendritic cells, which have been activated with the Dectin-1-specific antibody or fragments thereof alone or in combination with the other immune cells. For example, a combination therapy may include an antigen loadable antigen presenting cells (e.g., a dendritic cell) that have been activated with the Dectin-1-specific antibody or fragments thereof, a B cell and/or a T cell that together form the vaccine.

Herein disclosed are uses of agents that engage the Dectin-1 receptor on immune cells, alone or with co-activating agents, the combination activating antigen-presenting cells for therapeutic applications; use of a Dectin-1 binding agent linked to one or more antigens, with or without activating agents, on immune cells to make a vaccine; use of anti-Dectin-1 agents as co-activating agents of immune cells for the enhancement of immune responses directed through a cell surface receptor other than Dectin-1 expressed on immune cells; use of anti-Dectin-1 antibody V-region sequences capable of binding to and activating immune cells through the Dectin-1 receptor and/or use of Dectin-1 binding agents linked to one or more toxic agents for therapeutic purposes in the context of diseases known or suspected to result from inappropriate activation of immune cells via Dectin-1 or in the context of pathogenic cells or tissues that express Dectin-1.

Herein disclosed are modular rAb carriers that include a Dectin-1-specific antibody binding domain linked to one or more antigen carrier domains that comprise one half of a cohesin-dockerin binding pair. The antigen-specific binding domain may be at least a portion of an antibody and/or at least a portion of an antibody in a fusion protein with the one half of the cohesin-dockerin binding pair. The rAb may also be a complementary half of the cohesin-dockerin binding pair bound to an antigen that forms a complex with the modular rAb carrier and/or a complementary half of the cohesin-dockerin binding pair that is a fusion protein with an antigen. The antigen specific domain may be a full length antibody, an antibody variable region domain, an Fab fragment, a Fab' fragment, an F(ab)2 fragment, and Fv fragment, and Fabc fragment and/or a Fab fragment with portions of the Fc domain.

### BRIEF DESCRIPTION OF THE DRAWINGS

For a more complete understanding of the features and advantages of the present invention, reference is now made to the detailed description of the invention along with the accompanying figures and in which:

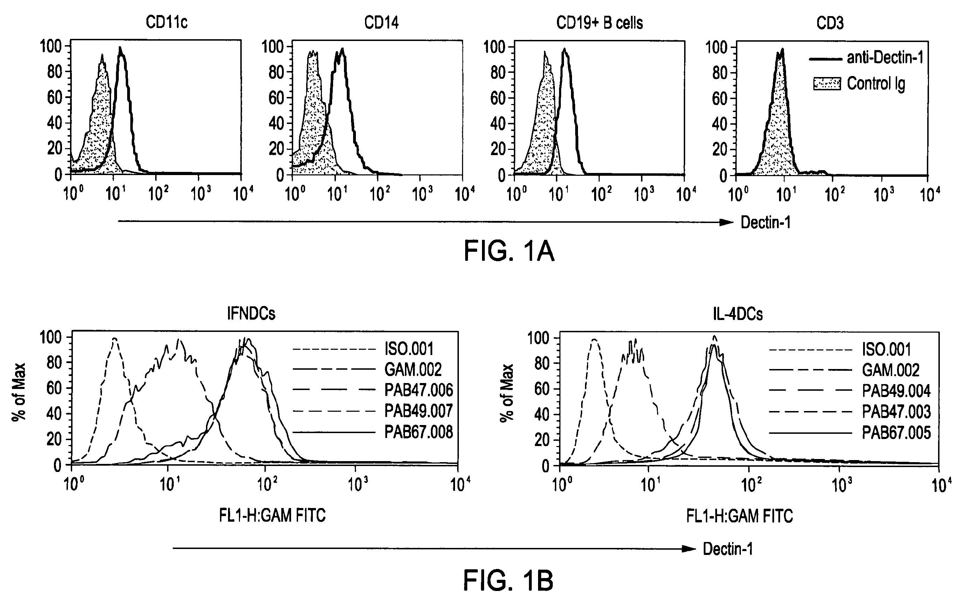

Figures 1A and 1B show the expression levels of Dectin-1 on PBMCs from normal donors;

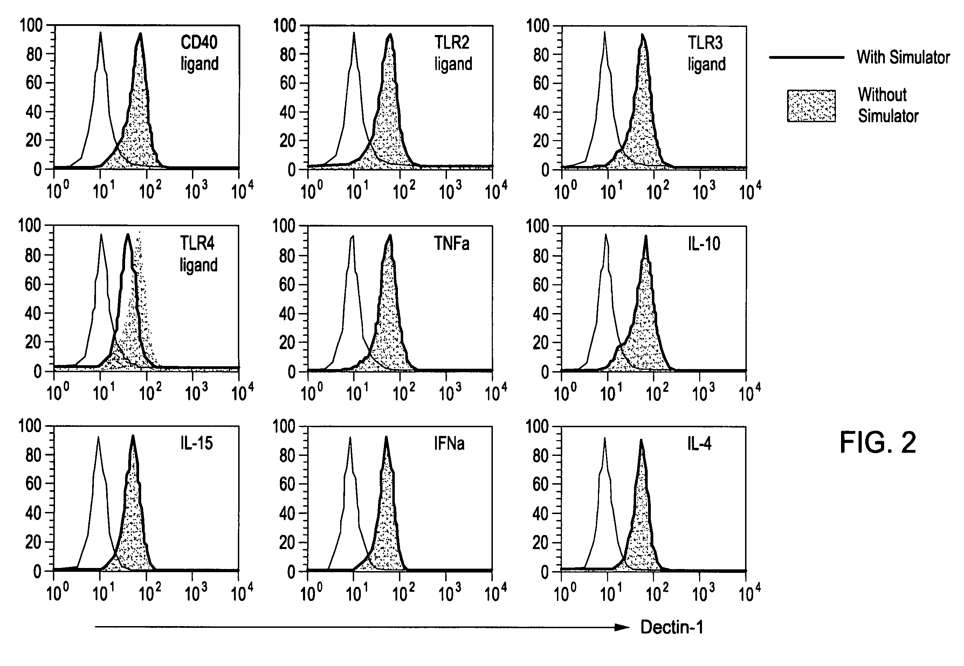

Figure 2 shows the results from in vitro-cultured IL-4DCs were cultured in the presence of CD40L (50 ng/ml), TLR2 ligand (100 ng/ml), TLR3 ligand (100 nM), TLR4 ligand (20 ng/ml), IL-10 (20 ng/ml), IL-15 (100 ng/ml), IFNα (500 U/ml), and IL-4 (50 ng/ml) for 24h;

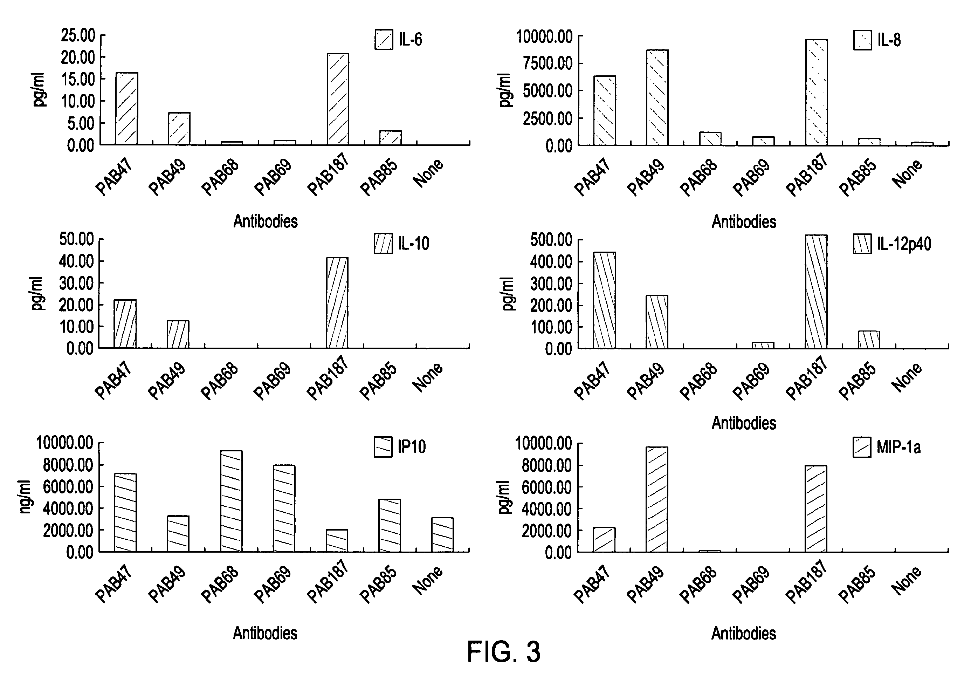

Figure 3 demonstrates that anti-Dectin-1 mAbs activate DCs;

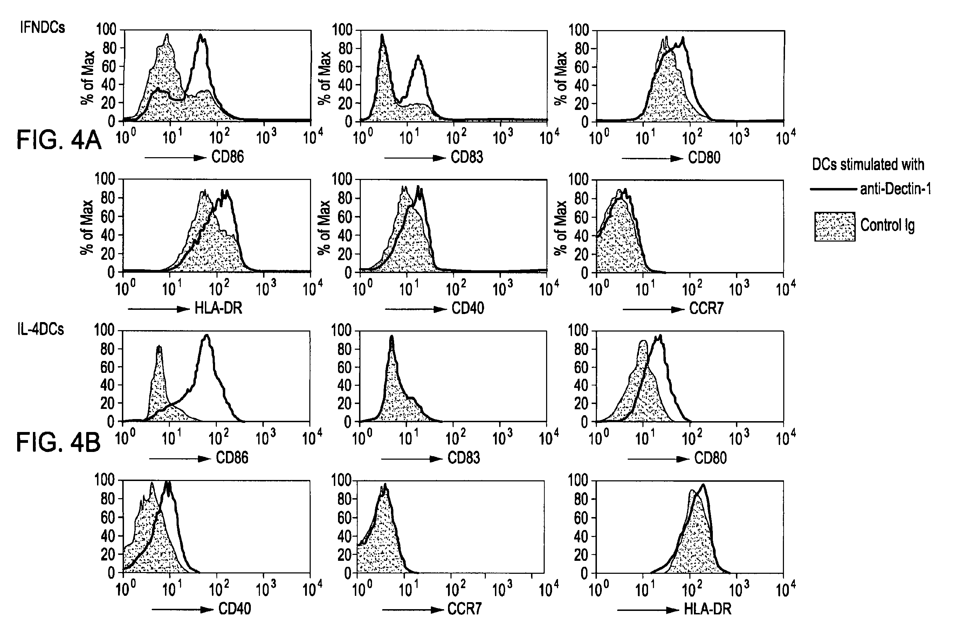

Figures 4A and 4B show that signaling through Dectin-1 can result in activation of both IL-4DCs and IFNDCs. Anti-Dectin-1 mAbs activate DCs. IFNDCs (Figure 4A) and IL-4DCs (Figure 4B) were stimulated with anti-Dectin-1 for 24 h, and then cells were stained with anti-CD86, 80, 40, 83, CCR7, and HLA-DR;

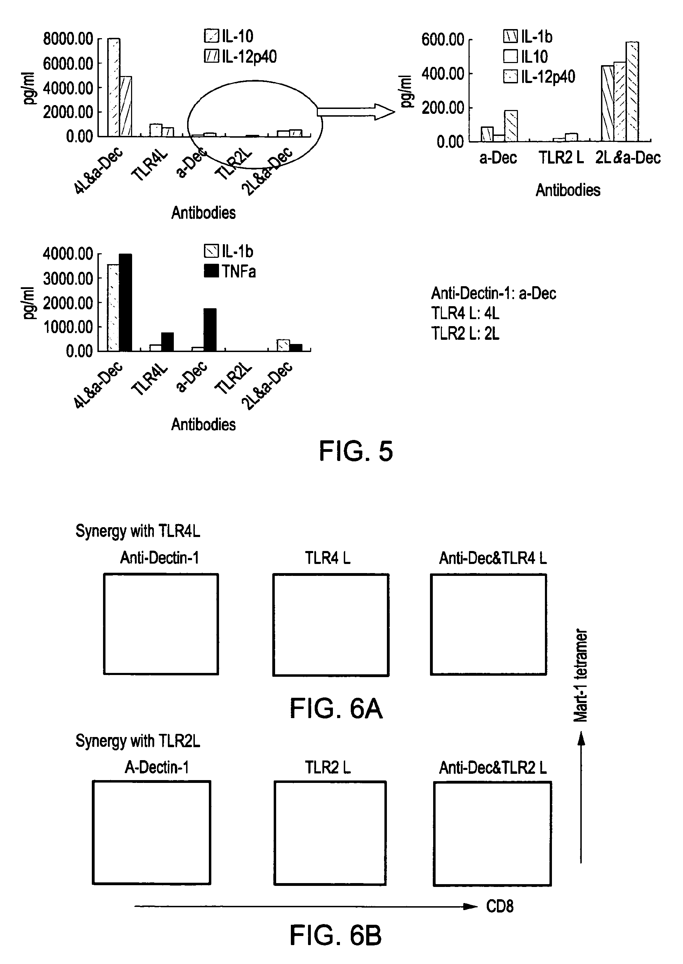

Figure 5 shows that Dectin-1 and TLRs synergize to activate DCs. IL-4DCs (2x10e5/200 ul/well) were cultured in 96-well plates coated with anti-Dectin-1 in the presence or absence of soluble TLR4 ligand (1.5 ng/ml) or TLR2 ligand (30 ng/ml) for 18 h. Control mAbs were also tested. After 18 h, culture supernatants were analyzed to measure cytokines and chemokines by Luminex;
Figures 6A and 6B show the results for IFNDCs (5000 DCs/well/200 uL) loaded with 10 uM Mart-1 peptide and activated with anti-Dectin-1 mAbs, TLR4 ligand, or anti-Dectin-1 mAbs and TLR4 ligand for 18 h (Figure 6A). Purified autologous CD8 T cells (2x10e5 cells/well/200 uL) were co-cultured in the presence of 20U/ml IL-2 and 10ng/ml IL-7 for 10 days. Cells were stained with anti-CD8 and Mart-1-HLA-A2-tetramer. Figure 6B shows the results for IFNDCs (5000 DCs/well/200 uL) were loaded with 10 uM Mart-1 peptide and activated with anti-Dectin-1 mAbs, TLR2 ligand, or anti-Dectin-1 mAbs and TLR2 ligand for 18 h. Purified autologous CD8 T cells (2x10⁵ cells/well/200 uL) were co-cultured in the presence of 20U/ml IL-2 and 10 ng/ml IL-7 for 10 days. Cells were stained with anti-CD8 and Mart-1-HLA-A2-tetramer;

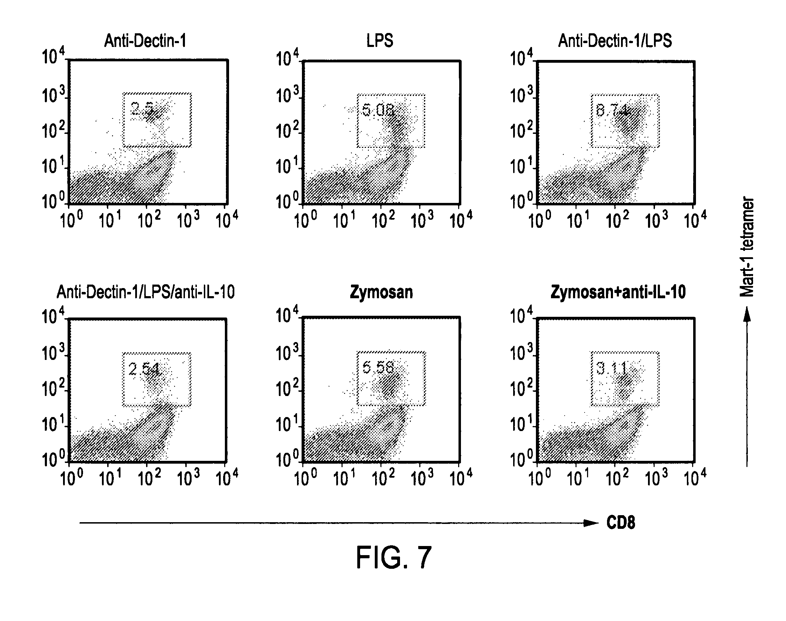

Figure 7 shows the results for IFNDCs (5000 DCs/well/200 uL) loaded with 10 uM Mart-1 peptide and activated with anti-Dectin-1 mAbs, TLR4 ligand, or anti-Dectin-1 mAbs and TLR4 ligand, Zymosan for 18 h. anti-IL-10 neutralizing antibody (5 ug/ml) was added into the DCs stimulated with anti-Dectin-1 and TLR4 ligand and Zymosan. Control antibody to anti-IL-10 was also tested, but there was no significant difference observed (data not shown). Purified autologous CD8 T cells (2x10e5 cells/well/200 uL) were co-cultured in the presence of 20U/ml IL-2 and 10ng/ml IL-7 for 10 days. Cells were stained with anti-CD8 and Mart-1-HLA-A2-tctramcr;

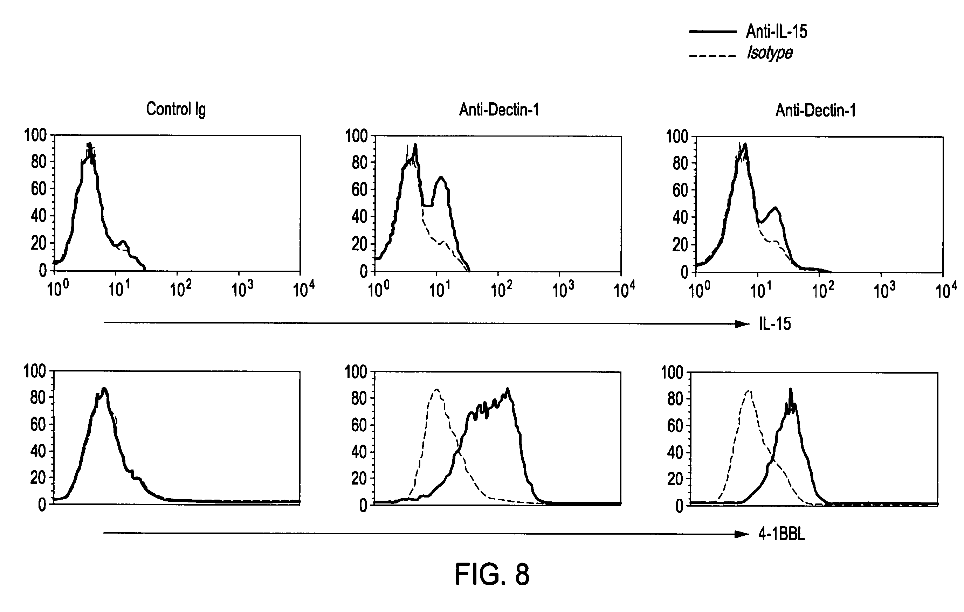

Figure 8 shows that DCs activated with anti-Dectin-1 mAbs express increased levels of IL-15 and 4-1BBL. IFNDCs (2x10⁵ /200 ul/well) were cultured in the 96-well plates coated with anti-Dectin-1 or control Ig for 18 h. After 18 h, cells were stained with anti-IL-15 and anti-4-1BBL;

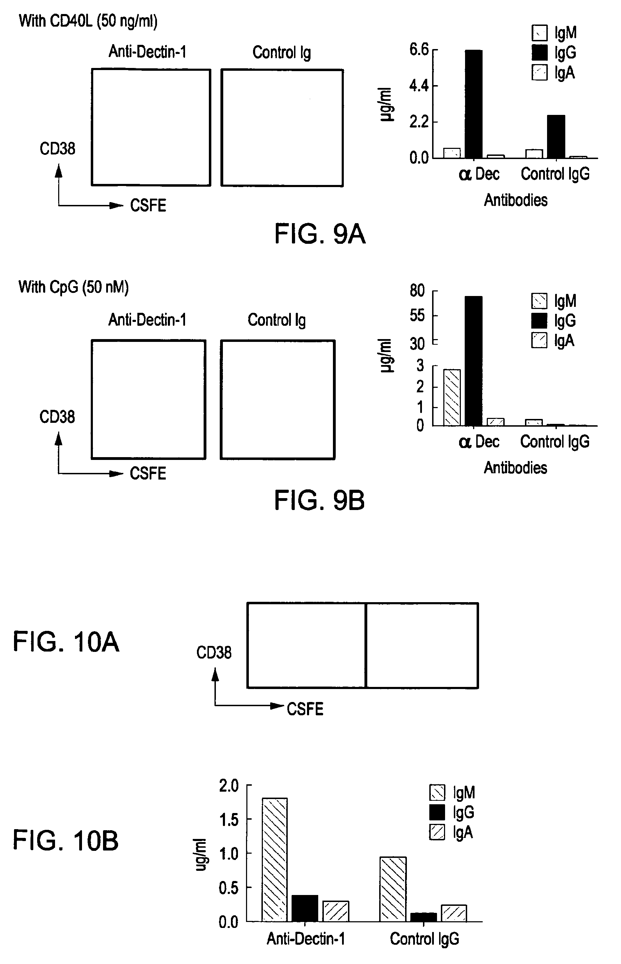

Figures 9A and 9B show that Dectin-1 expressed on DCs contributes to enhanced humoral immune responses. 6-day IL-4 DCs, 5x10³/well, were incubated in 96 well plates coated with anti-Dectin-1 or control mAbs for 16-18 h, and then 2x10e4 autologous CD19⁺ B cells stained with CFSE were co-cultured in the presence of 20 U/ml IL-2 and 50 ng/ml CD40L (Figure 9A) or 50 nM CpG (Figure 9B). On day six, cells were stained with fluorescently labeled anti-CD38 antibody. Culture supernatants on day thirteen were analyzed for total immunoglobulins by sandwich ELISA;
Figures 10A and 10B show that Dectin-1 expressed on B cells contributes to B cell activation and immunoglobulin production. Figure 10A. Purified CD19+ B cells (20000 cells/well/200ul) were cultured in plates coated with the mAbs, either anti-Dectin-1 or control Ig, in the presence of 50 nM CpG and 20U/ml IL-2 for 6 days. Cells were stained with anti-CD38 and cell proliferation was measured by CFSE dilution. Figure 10B. On day 13 of the culture, culture supernatants were analyzed for measuring total immunoglobulins produced by ELISA;

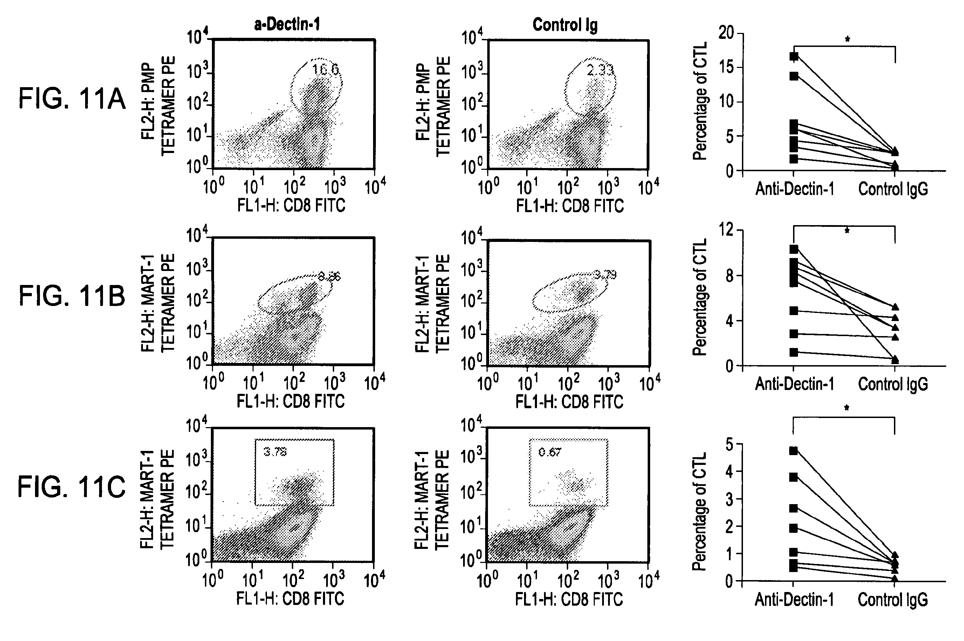

Figures 11A to 11C show that Dectin-1 expressed on DCs contributes to enhanced cellular immune responses. Figure 11A. 5x10³ of IFNDCs were cultured in plates coated with anti-Dectin-1 or control mAbs in the presence of 100 nM of recombinant Flu M1 protein for 16-18 h, and then purified autologous CD8 T cells were co-cultured in the presence of 20 U/ml IL-2 and 10ng/ml of IL-7 for 7 days. Cells were stained with anti-CD8 and tetramer for Flu M1 peptide epitope for HLA-A2. Figure 11B and Figure 11C. IFNDCs were loaded with 10 uM of Mart-1 peptide (Figure 11B) or 100 nM Coh-Mart-1 recombinant protein, and then stimulated with anti-Dectin-1 or control antibodies as described in Figure 11A. After 10-day culture, cells were stained with anti-CD8 and tetramer (HLA-A2-mart-1 peptide). Each line represents data generated from an individual donor. All cells used in this study were from HLA-A201 positive normal donors;

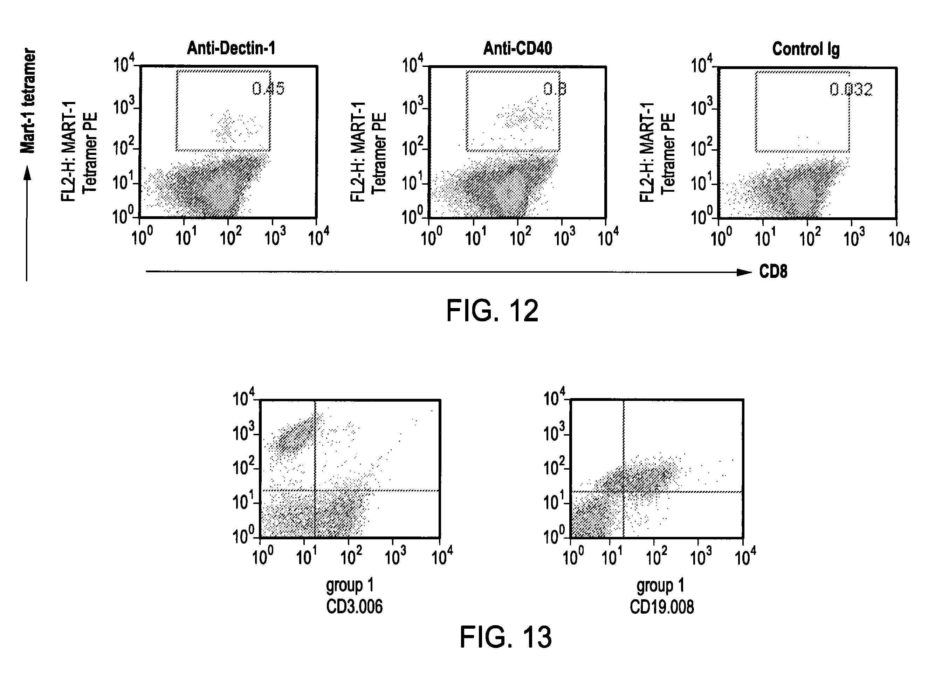

Figure 12 shows that anti-Dectin-1 mAbs in soluble form activate DCs to enhance Mart-1 specific CD8 T cell priming. 5x103 of IFNDCs were cultured in the presence of 0.5 ug/ml of anti-Dectin-1, anti-CD40, or control mAb for 16 h. Before purified autologous T cells (2x10⁵/well) were added into the culture, 20 uM Mart-1 peptide were added. After 10 days, cells were stained with anti-CD8 and tetramer (HLA-A2-mart-1 peptide). All cells used in this study were from HLA-A201 positive normal donors;
Figure 13 shows that tonsils from normal donors were acquired and a cell suspension was prepared using DNAse-free Collagenase type III. Cells were washed vigorously with PBS containing 5% FCS, and then stained with anti-CD3 or anti-CD19 with anti-Dectin-1 mAbs. isotype-matched control mAbs were used;

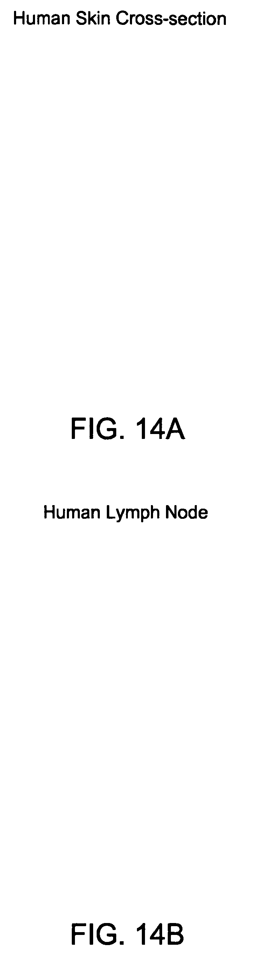

Figures 14A and 14B show tissue sections of human skin (Figure 14A) and lymph node (Figure 14B) from normal donors were prepared and stained with DAPI (Blue) and anti-Dectin-1 (Red) prepared in this study;

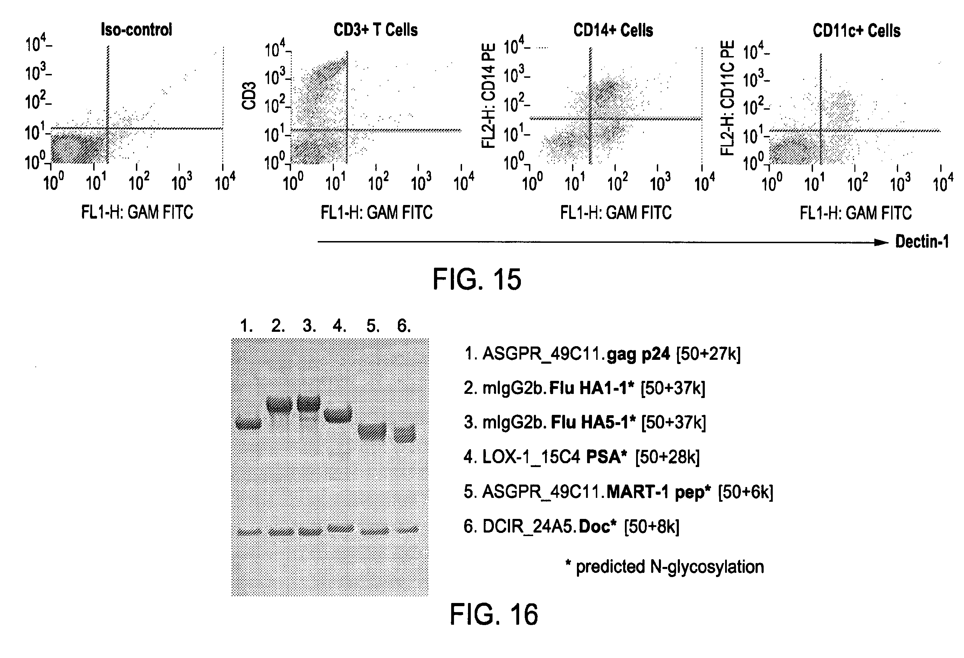

Figure 15 shows that PBMC from non-human primates (Cynomolgus) were stained with anti-Dectin-1 mAb and antibodies to cell surface markers;
Figure 16 shows reduced SDS-PAGE analysis of typical rAb.antigen fusion proteins - bearing many of the antigens shown or mentioned above.

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The present invention also includes a recombinant humanized mAb (directed to the specific human dendritic cell receptor Dectin-1) fused through the Ab heavy chain to antigens known or suspected to encode protective antigens. These include as examples for vaccination against various agents - hemagglutinins from Influenza H5N1; HIV gag from attenuated toxins from Ricin, Anthrax toxin, and Staphylococcus B enterotoxin; 'strings' of antigenic peptides from melanona anigens, etc. The present invention will find use in preventative or therapeutic vaccination of at-risk or infected people. The invention has broad application for vaccination against many diseases and cancers, both for human and animal use.

As used herein, the term "modular rAb carrier" is used to describe a recombinant antibody system that has been engineered to provide the controlled modular addition of diverse antigens, activating proteins, or other antibodies to a single recombinant monoclonal antibody (mAb), in this case, an anti-Dectin-1 monoclonal antibody. The rAb may be a monoclonal antibody made using standard hybridoma techniques, recombinant antibody display, humanized monoclonal antibodies and the like. The modular rAb carrier can be used to, e.g., target (via one primary recombinant antibody against an internalizing receptor, e.g., a human dendritic cell receptor) multiple antigens and/or antigens and an activating cytokine to dendritic cells (DC). The modular rAb carrier may also be used to join two different recombinant mAbs end-to-end in a controlled and defined manner.

The antigen binding portion of the "modular rAb carrier" may be one or more variable domains, one or more variable and the first constant domain, an Fab fragment, a Fab' fragment, an F(ab)₂ fragment, and Fv fragment, and Fabc fragment and/or a Fab fragment with portions of the Fc domain to which the cognate modular binding portions are added to the amino acid sequence and/or bound. The antibody for use in the modular rAb carrier can be of any isotype or class, subclass or from any source (animal and/or recombinant).

In one non-limiting example, the modular rAb carrier is engineered to have one or more modular cohesin-dockerin protein domains for making specific and defined protein complexes in the context of engineered recombinant mAbs. The mAb is a portion of a fusion protein that includes one or more modular cohesin-dockerin protein domains carboxy from the antigen binding domains of the mAb. The cohesin-dockerin protein domains may even be attached post-translationally, e.g., by using chemical cross-linkers and/or disulfide bonding.

The term "antigen" as used herein refers to a molecule that can initiate a humoral and/or cellular immune response in a recipient of the antigen. Antigen may be used in two different contexts with the present invention: as a target for the antibody or other antigen recognition domain of the rAb or as the molecule that is carried to and/or into a cell or target by the rAb as part of a dockerin/cohesin-molecule complement to the modular rAb carrier. The antigen is usually an agent that causes a disease for which a vaccination would be advantageous treatment. When the antigen is presented on MHC, the peptide is often about 8 to about 25 amino acids. Antigens include any type of biologic molecule, including, for example, simple intermediary metabolites, sugars, lipids and hormones as well as macromolecules such as complex carbohydrates, phospholipids, nucleic acids and proteins. Common categories of antigens include, but are not limited to, viral antigens, bacterial antigens, fungal antigens, protozoal and other parasitic antigens, tumor antigens, antigens involved in autoimmune disease, allergy and graft rejection, and other miscellaneous antigens.

The modular rAb carrier is able to carry any number of active agents, e.g., antibiotics, anti-infective agents, antiviral agents, anti-tumoral agents, antipyretics, analgesics, anti-inflammatory agents, therapeutic agents for osteoporosis, enzymes, cytokines, anticoagulants, polysaccharides, collagen, cells, and combinations of two or more of the foregoing active agents. Examples of antibiotics for delivery using the present invention include, without limitation, tetracycline, aminoglycosides, penicillins, cephalosporins, sulfonamide drugs, chloramphenicol sodium succinate, erythromycin, vancomycin, lincomycin, clindamycin, nystatin, amphotericin B, amantidine, idoxuridine, p-amino salicyclic acid, isoniazid, rifampin, antinomycin D, mithramycin, daunomycin, adriamycin, bleomycin, vinblastine, vincristine, procarbazine, imidazole carboxamide, and the like.

Examples of anti-tumor agents for delivery using the present invention include, without limitation, doxorubicin, Daunorubicin, taxol, methotrexate, and the like. Examples of antipyretics and analgesics include aspirin, Motrin®, Ibuprofen®, naprosyn, acetaminophen, and the like.

Examples of anti-inflammatory agents for delivery using the present invention include, without limitation, include NSAIDS, aspirin, steroids, dexamethasone, hydrocortisone, prednisolone, Diclofenac Na, and the like.

Examples of therapeutic agents for treating osteoporosis and other factors acting on bone and skeleton include for delivery using the present invention include, without limitation, calcium, alendronate, bone GLa peptide, parathyroid hormone and its active fragments, histone H4-related bone formation and proliferation peptide and mutations, derivatives and analogs thereof.

Examples of enzymes and enzyme cofactors for delivery using the present invention include, without limitation, pancrease, L-asparaginase, hyaluronidase, chymotrypsin, trypsin, tPA, streptokinase, urokinase, pancreatin, collagenase, trypsinogen, chymotrypsinogen, plasminogen, streptokinase, adenyl cyclase, superoxide dismutase (SOD), and the like.

Examples of cytokines for delivery using the present invention include, without limitation, interleukins, transforming growth factors (TGFs), fibroblast growth factors (FGFs), platelet derived growth factors (PDGFs), epidermal growth factors (EGFs), connective tissue activated peptides (CTAPs), osteogenic factors, and biologically active analogs, fragments, and derivatives of such growth factors. Cytokines may be B/T-cell differentiation factors, B/T-cell growth factors, mitogenic cytokines, chemotactic cytokines, colony stimulating factors, angiogenesis factors, IFN-α, IFN-β, IFN-γ, IL1, IL2, IL3, IL4, IL5, IL6, IL7, IL8, IL9, IL10, IL11, IL12, IL 13, IL 14, IL15, IL16, IL 17, IL 18, etc., leptin, myostatin, macrophage stimulating protein, platelet-derived growth factor, TNF-α, TNF-β, NGF, CD40L, CD137L/4-1BBL, human lymphotoxin-β, G-CSF, M-CSF, GM-CSF, PDGF, IL-1α, IL1- β, IP-10, PF4, GRO, 9E3, erythropoietin, endostatin, angiostatin, VEGF or any fragments or combinations thereof. Other cytokines include members of the transforming growth factor (TGF) supergene family include the beta transforming growth factors (for example TGF-β1, TGF-β2, TGF-β3); bone morphogenetic proteins (for example, BMP-1, BMP-2, BMP-3, BMP-4, BMP-5, BMP-6, BMP-7, BMP-8, BMP-9); heparin-binding growth factors (for example, fibroblast growth factor (FGF), epidermal growth factor (EGF), platelet-derived growth factor (PDGF), insulin-like growth factor (IGF)); Inhibins (for example, Inhibin A, Inhibin B); growth differentiating factors (for example, GDF-1); and Activins (for example, Activin A, Activin B, Activin AB).

Examples of growth factors for delivery using the present invention include, without limitation, growth factors that can be isolated from native or natural sources, such as from mammalian cells, or can be prepared synthetically, such as by recombinant DNA techniques or by various chemical processes. In addition, analogs, fragments, or derivatives of these factors can be used, provided that they exhibit at least some of the biological activity of the native molecule. For example, analogs can be prepared by expression of genes altered by site-specific mutagenesis or other genetic engineering techniques.

Examples of anticoagulants for delivery using the present invention include, without limitation, include warfarin, heparin, Hirudin, and the like. Examples of factors acting on the immune system include for delivery using the present invention include, without limitation, factors which control inflammation and malignant neoplasms and factors which attack infective microorganisms, such as chemotactic peptides and bradykinins.

Examples of viral antigens include, but are not limited to, e.g., retroviral antigens such as retroviral antigens from the human immunodeficiency virus (HIV) antigens such as gene products of the gag, pol, and env genes, the Nef protein, reverse transcriptase, and other HIV components; hepatitis viral antigens such as the S, M, and L proteins of hepatitis B virus, the pre-S antigen of hepatitis B virus, and other hepatitis, e.g., hepatitis A, B, and C, viral components such as hepatitis C viral RNA; influenza viral antigens such as hemagglutinin and neuraminidase and other influenza viral components; measles viral antigens such as the measles virus fusion protein and other measles virus components; rubella viral antigens such as proteins E1 and E2 and other rubella virus components; rotaviral antigens such as VP7sc and other rotaviral components; cytomegaloviral antigens such as envelope glycoprotein B and other cytomegaloviral antigen components; respiratory syncytial viral antigens such as the RSV fusion protein, the M2 protein and other respiratory syncytial viral antigen components; herpes simplex viral antigens such as immediate early proteins, glycoprotein D, and other herpes simplex viral antigen components; varicella zoster viral antigens such as gpI, gpII, and other varicella zoster viral antigen components; Japanese encephalitis viral antigens such as proteins E, M-E, M-E-NS1, NS1, NS1-NS2A, 80% E, and other Japanese encephalitis viral antigen components; rabies viral antigens such as rabies glycoprotein, rabies nucleoprotein and other rabies viral antigen components. See Fundamental Virology, Second Edition, eds. Fields, B. N. and Knipe, D. M. (Raven Press, New York, 1991) for additional examples of viral antigens.

Antigenic targets that may be delivered using the rAb-DC/DC-antigen vaccines of the present invention include genes encoding antigens such as viral antigens, bacterial antigens, fungal antigens or parasitic antigens. Viruses include picornavirus, coronavirus, togavirus, flavirvirus, rhabdovirus, paramyxovirus, orthomyxovirus, bunyavirus, arenavirus, reovirus, retrovirus, papilomavirus, parvovirus, herpesvirus, poxvirus, hepadnavirus, and spongiform virus. Other viral targets include influenza, herpes simplex virus 1 and 2, measles, dengue, smallpox, polio or HIV. Pathogens include trypanosomes, tapeworms, roundworms, helminthes, malaria. Tumor markers, such as fetal antigen or prostate specific antigen, may be targeted in this manner. Other examples include: HIV env proteins and hepatitis B surface antigen. Administration of a vector according to the present invention for vaccination purposes would require that the vector-associated antigens be sufficiently non-immunogenic to enable long term expression of the transgene, for which a strong immune response would be desired. In some cases, vaccination of an individual may only be required infrequently, such as yearly or biennially, and provide long term immunologic protection against the infectious agent. Specific examples of organisms, allergens and nucleic and amino sequences for use in vectors and ultimately as antigens with the present invention may be found in U.S. Patent No. 6,541,011, relevant portions incorporated herein by reference, in particular, the tables that match organisms and specific sequences that may be used with the present invention.

Bacterial antigens for use with the rAb vaccine disclosed herein include, but are not limited to, e.g., bacterial antigens such as pertussis toxin, filamentous hemagglutinin, pertactin, FIM2, FIM3, adenylate cyclase and other pertussis bacterial antigen components; diptheria bacterial antigens such as diptheria toxin or toxoid and other diptheria bacterial antigen components; tetanus bacterial antigens such as tetanus toxin or toxoid and other tetanus bacterial antigen components; streptococcal bacterial antigens such as M proteins and other streptococcal bacterial antigen components; gram-negative bacilli bacterial antigens such as lipopolysaccharides and other gram-negative bacterial antigen components, Mycobacterium tuberculosis bacterial antigens such as mycolic acid, heat shock protein 65 (HSP65), the 30 kDa major secreted protein, antigen 85A and other mycobacterial antigen components; Helicobacter pylori bacterial antigen components; pneumococcal bacterial antigens such as pneumolysin, pneumococcal capsular polysaccharides and other pneumococcal bacterial antigen components; haemophilus influenza bacterial antigens such as capsular polysaccharides and other haemophilus influenza bacterial antigen components; anthrax bacterial antigens such as anthrax protective antigen and other anthrax bacterial antigen components; rickettsiae bacterial antigens such as rompA and other rickettsiae bacterial antigen component. Also included with the bacterial antigens described herein are any other bacterial, mycobacterial, mycoplasmal, rickettsial, or chlamydial antigens. Partial or whole pathogens may also be: haemophilus influenza; Plasmodium falciparum; neisseria meningitidis; streptococcus pneumoniae; neisseria gonorrhoeae; salmonella serotype typhi; shigella; vibrio cholerae; Dengue Fever; Encephalitides; Japanese Encephalitis; lyme disease; Yersinia pestis; west nile virus; yellow fever; tularemia; hepatitis (viral; bacterial); RSV (respiratory syncytial virus); HPIV 1 and HPIV 3; adenovirus; small pox; allergies and cancers.

Fungal antigens for use with compositions and methods of the invention include, but are not limited to, e.g., candida fungal antigen components; histoplasma fungal antigens such as heat shock protein 60 (HSP60) and other histoplasma fungal antigen components; cryptococcal fungal antigens such as capsular polysaccharides and other cryptococcal fungal antigen components; coccidiodes fungal antigens such as spherule antigens and other coccidiodes fungal antigen components; and tinea fungal antigens such as trichophytin and other coccidiodes fungal antigen components.

Examples of protozoal and other parasitic antigens include, but are not limited to, e.g., plasmodium falciparum antigens such as merozoite surface antigens, sporozoite surface antigens, circumsporozoite antigens, gametocyte/gamete surface antigens, blood-stage antigen pf 155/RESA and other plasmodial antigen components; toxoplasma antigens such as SAG-1, p30 and other toxoplasmal antigen components; schistosomae antigens such as glutathione-S-transferase, paramyosin, and other schistosomal antigen components; leishmania major and other leishmaniae antigens such as gp63, lipophosphoglycan and its associated protein and other leishmanial antigen components; and trypanosoma cruzi antigens such as the 75-77 kDa antigen, the 56 kDa antigen and other trypanosomal antigen components.

Antigen that can be targeted using the rAb of the present invention will generally be selected based on a number of factors, including: likelihood of internalization, level of immune cell specificity, type of immune cell targeted, level of immune cell maturity and/or activation and the like. Examples of cell surface markers for dendritic cells include, but are not limited to, MHC class I, MHC Class II, B7-2, CD18, CD29, CD31, CD43, CD44, CD45, CD54, CD58, CD83, CD86, CMRF-44, CMRF-56, DCIR and/or Dectin-1 and the like; while in some cases also having the absence of CD2, CD3, CD4, CD8, CD14, CD15, CD16, CD 19, CD20, CD56, and/or CD57. Examples of cell surface markers for antigen presenting cells include, but are not limited to, MHC class I, MHC Class II, CD40, CD45, B7-1, B7-2, IFN-γ receptor and IL-2 receptor, ICAM-1 and/or Fcγ receptor. Examples of cell surface markers for T cells include, but are not limited to, CD3, CD4, CD8, CD 14, CD20, CD11b, CD16, CD45 and HLA-DR.

Target antigens on cell surfaces for delivery includes those characteristic of tumor antigens typically will be derived from the cell surface, cytoplasm, nucleus, organelles and the like of cells of tumor tissue. Examples of tumor targets for the antibody portion of the present invention include, without limitation, hematological cancers such as leukemias and lymphomas, neurological tumors such as astrocytomas or glioblastomas, melanoma, breast cancer, lung cancer, head and neck cancer, gastrointestinal tumors such as gastric or colon cancer, liver cancer, pancreatic cancer, genitourinary tumors such cervix, uterus, ovarian cancer, vaginal cancer, testicular cancer, prostate cancer or penile cancer, bone tumors, vascular tumors, or cancers of the lip, nasopharynx, pharynx and oral cavity, esophagus, rectum, gall bladder, biliary tree, larynx, lung and bronchus, bladder, kidney, brain and other parts of the nervous system, thyroid, Hodgkin's disease, non-Hodgkin's lymphoma, multiple myeloma and leukemia.

Examples of antigens that may be delivered alone or in combination to immune cells for antigen presentation using the present invention includes tumor proteins, e.g., mutated oncogenes; viral proteins associated with tumors; and tumor mucins and glycolipids. The antigens may be viral proteins associated with tumors would be those from the classes of viruses noted above. Certain antigens may be characteristic of tumors (one subset being proteins not usually expressed by a tumor precursor cell), or may be a protein which is normally expressed in a tumor precursor cell, but having a mutation characteristic of a tumor. Other antigens include mutant variant(s) of the normal protein having an altered activity or subcellular distribution, e.g., mutations of genes giving rise to tumor antigens.

Specific non-limiting examples of tumor antigens include: CEA, prostate specific antigen (PSA), HER-2/neu, BAGE, GAGE, MAGE 1-4, 6 and 12, MUC (Mucin) (e.g., MUC-1, MUC-2, etc.), GM2 and GD2 gangliosides, ras, myc, tyrosinase, MART (melanoma antigen), Pmel 17(gp100), GnT-V intron V sequence (N-acetylglucoaminyltransferase V intron V sequence), Prostate Ca psm, PRAME (melanoma antigen), β-catenin, MUM-1-B (melanoma ubiquitous mutated gene product), GAGE (melanoma antigen) 1, BAGE (melanoma antigen) 2-10, c-ERB2 (Her2/neu), EBNA (Epstein-Barr Virus nuclear antigen) 1-6, gp75, human papilloma virus (HPV) E6 and E7, p53, lung resistance protein (LRP), Bcl-2, and Ki-67. In addition, the immunogenic molecule can be an autoantigen involved in the initiation and/or propagation of an autoimmune disease, the pathology of which is largely due to the activity of antibodies specific for a molecule expressed by the relevant target organ, tissue, or cells, e.g., SLE or MG. In such diseases, it can be desirable to direct an ongoing antibody-mediated (i.e., a Th2-type) immune response to the relevant autoantigen towards a cellular (i.e., a Th1-type) immune response. Alternatively, it can be desirable to prevent onset of or decrease the level of a Th2 response to the autoantigen in a subject not having, but who is suspected of being susceptible to, the relevant autoimmune disease by prophylactically inducing a Th1 response to the appropriate autoantigen. Autoantigens of interest include, without limitation: (a) with respect to SLE, the Smith protein, RNP ribonucleoprotein, and the SS-A and SS-B proteins; and (b) with respect to MG, the acetylcholine receptor. Examples of other miscellaneous antigens involved in one or more types of autoimmune response include, e.g., endogenous hormones such as luteinizing hormone, follicular stimulating hormone, testosterone, growth hormone, prolactin, and other hormones.

Antigens involved in autoimmune diseases, allergy, and graft rejection can be used in the compositions and methods of the invention. For example, an antigen involved in any one or more of the following autoimmune diseases or disorders can be used in the present invention: diabetes, diabetes mellitus, arthritis (including rheumatoid arthritis, juvenile rheumatoid arthritis, osteoarthritis, psoriatic arthritis), multiple sclerosis, myasthenia gravis, systemic lupus erythematosis, autoimmune thyroiditis, dermatitis (including atopic dermatitis and eczematous dermatitis), psoriasis, Sjogren's Syndrome, including keratoconjunctivitis sicca secondary to Sjogren's Syndrome, alopecia areata, allergic responses due to arthropod bite reactions, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Crohn's disease, Graves ophthalmopathy, sarcoidosis, primary biliary cirrhosis, uveitis posterior, and interstitial lung fibrosis. Examples of antigens involved in autoimmune disease include glutamic acid decarboxylase 65 (GAD 65), native DNA, myelin basic protein, myelin proteolipid protein, acetylcholine receptor components, thyroglobulin, and the thyroid stimulating hormone (TSH) receptor. Examples of antigens involved in allergy include pollen antigens such as Japanese cedar pollen antigens, ragweed pollen antigens, rye grass pollen antigens, animal derived antigens such as dust mite antigens and feline antigens, histocompatiblity antigens, and penicillin and other therapeutic drugs. Examples of antigens involved in graft rejection include antigenic components of the graft to be transplanted into the graft recipient such as heart, lung, liver, pancreas, kidney, and neural graft components. The antigen may be an altered peptide ligand useful in treating an autoimmune disease.

As used herein, the term "epitope(s)" refer to a peptide or protein antigen that includes a primary, secondary or tertiary structure similar to an epitope located within any of a number of pathogen polypeptides encoded by the pathogen DNA or RNA. The level of similarity will generally be to such a degree that monoclonal or polyclonal antibodies directed against such polypeptides will also bind to, react with, or otherwise recognize, the peptide or protein antigen. Various immunoassay methods may be employed in conjunction with such antibodies, such as, for example, Western blotting, ELISA, RIA, and the like, all of which are known to those of skill in the art. The identification of pathogen epitopes, and/or their functional equivalents, suitable for use in vaccines is part of the present invention. Once isolated and identified, one may readily obtain functional equivalents. For example, one may employ the methods of Hopp, as taught in U.S. Pat. No. 4,554,101, incorporated herein by reference, which teaches the identification and preparation of epitopes from amino acid sequences on the basis of hydrophilicity. The methods described in several other papers, and software programs based thereon, can also be used to identify epitopic core sequences (see, for example, Jameson and Wolf, 1988; Wolf et al., 1988; U.S. Pat. No. 4,554,101). The amino acid sequence of these "epitopic core sequences" may then be readily incorporated into peptides, either through the application of peptide synthesis or recombinant technology.

The preparation of vaccine compositions that includes the nucleic acids that encode antigens of the invention as the active ingredient, may be prepared as injectables, either as liquid solutions or suspensions; solid forms suitable for solution in, or suspension in, liquid prior to infection can also be prepared. The preparation may be emulsified, encapsulated in liposomes. The active immunogenic ingredients are often mixed with carriers which are pharmaceutically acceptable and compatible with the active ingredient.

The term "pharmaceutically acceptable carrier" refers to a carrier that does not cause an allergic reaction or other untoward effect in subjects to whom it is administered. Suitable pharmaceutically acceptable carriers include, for example, one or more of water, saline, phosphate buffered saline, dextrose, glycerol, ethanol, or the like and combinations thereof. In addition, if desired, the vaccine can contain minor amounts of auxiliary substances such as wetting or emulsifying agents, pH buffering agents, and/or adjuvants which enhance the effectiveness of the vaccine. Examples of adjuvants that may be effective include but are not limited to: aluminum hydroxide, N-acetyl-muramyl-L-threonyl-D-isoglutamine (thr-MDP), N-acetyl-nor-muramyl-L-alanyl-D-isoglutamine, MTP-PE and RIBI, which contains three components extracted from bacteria, monophosporyl lipid A, trehalose dimycolate and cell wall skeleton (MPL+TDM+CWS) in a 2% squalene/Tween 80 emulsion. Other examples of adjuvants include DDA (dimethyldioctadecylammonium bromide), Freund's complete and incomplete adjuvants and QuilA. In addition, immune modulating substances such as lymphokines (e.g., IFN-γ, IL-2 and IL-12) or synthetic IFN-γ inducers such as poly I:C can be used in combination with adjuvants described herein.

Pharmaceutical products that may include a naked polynucleotide with a single or multiple copies of the specific nucleotide sequences that bind to specific DNA-binding sites of the apolipoproteins present on plasma lipoproteins as described in the current invention. The polynucleotide may encode a biologically active peptide, antisense RNA, or ribozyme and will be provided in a physiologically acceptable administrable form. Another pharmaceutical product that may spring from the current invention may include a highly purified plasma lipoprotein fraction, isolated according to the methodology, described herein from either the patients blood or other source, and a polynucleotide containing single or multiple copies of the specific nucleotide sequences that bind to specific DNA-binding sites of the apolipoproteins present on plasma lipoproteins, prebound to the purified lipoprotein fraction in a physiologically acceptable, administrable form.

Yet another pharmaceutical product may include a highly purified plasma lipoprotein fraction which contains recombinant apolipoprotein fragments containing single or multiple copies of specific DNA-binding motifs, prebound to a polynucleotide containing single or multiple copies of the specific nucleotide sequences, in a physiologically acceptable administrable form. Yet another pharmaceutical product may include a highly purified plasma lipoprotein fraction which contains recombinant apolipoprotein fragments containing single or multiple copies of specific DNA-binding motifs, prebound to a polynucleotide containing single or multiple copies of the specific nucleotide sequences, in a physiologically acceptable administrable form.

The dosage to be administered depends to a great extent on the body weight and physical condition of the subject being treated as well as the route of administration and frequency of treatment. A pharmaceutical composition that includes the naked polynucleotide prebound to a highly purified lipoprotein fraction may be administered in amounts ranging from 1 µg to 1 mg polynucleotide and 1 µg to 100 mg protein.

Administration of an rAb and rAb complexes a patient will follow general protocols for the administration of chemotherapeutics, taking into account the toxicity, if any, of the vector. It is anticipated that the treatment cycles would be repeated as necessary. It also is contemplated that various standard therapies, as well as surgical intervention, may be applied in combination with the described gene therapy.

Where clinical application of a gene therapy is contemplated, it will be necessary to prepare the complex as a pharmaceutical composition appropriate for the intended application. Generally this will entail preparing a pharmaceutical composition that is essentially free of pyrogens, as well as any other impurities that could be harmful to humans or animals. One also will generally desire to employ appropriate salts and buffers to render the complex stable and allow for complex uptake by target cells.

Aqueous compositions of the present invention may include an effective amount of the compound, dissolved or dispersed in a pharmaceutically acceptable carrier or aqueous medium. Such compositions can also be referred to as inocula. The use of such media and agents for pharmaceutical active substances is well known in the art. Except insofar as any conventional media or agent is incompatible with the active ingredient, its use in the therapeutic compositions is contemplated. Supplementary active ingredients also can be incorporated into the compositions. The compositions of the present invention may include classic pharmaceutical preparations. Dispersions also can be prepared in glycerol, liquid polyethylene glycols, and mixtures thereof and in oils. Under ordinary conditions of storage and use, these preparations contain a preservative to prevent the growth of microorganisms.

Disease States. Depending on the particular disease to be treated, administration of therapeutic compositions according to the present invention will be via any common route so long as the target tissue is available via that route in order to maximize the delivery of antigen to a site for maximum (or in some cases minimum) immune response. Administration will generally be by orthotopic, intradermal, subcutaneous, intramuscular, intraperitoneal or intravenous injection. Other areas for delivery include: oral, nasal, buccal, rectal, vaginal or topical. Topical administration would be particularly advantageous for treatment of skin cancers. Such compositions would normally be administered as pharmaceutically acceptable compositions that include physiologically acceptable carriers, buffers or other excipients.

Vaccine or treatment compositions of the invention may be administered parenterally, by injection, for example, either subcutaneously or intramuscularly. Additional formulations which are suitable for other modes of administration include suppositories, and in some cases, oral formulations or formulations suitable for distribution as aerosols. In the case of the oral formulations, the manipulation of T-cell subsets employing adjuvants, antigen packaging, or the addition of individual cytokines to various formulation that result in improved oral vaccines with optimized immune responses. For suppositories, traditional binders and carriers may include, for example, polyalkylene glycols or triglycerides; such suppositories may be formed from mixtures containing the active ingredient in the range of 0.5% to 10%, preferably 1%-2%. Oral formulations include such normally employed excipients as, for example, pharmaceutical grades of mannitol, lactose, starch magnesium stearate, sodium saccharine, cellulose, magnesium carbonate, and the like. These compositions take the form of solutions, suspensions, tablets, pills, capsules, sustained release formulations or powders and contain 10%-95% of active ingredient, preferably 25-70%.

The antigen encoding nucleic acids of the invention may be formulated into the vaccine or treatment compositions as neutral or salt forms. Pharmaceutically acceptable salts include the acid addition salts (formed with free amino groups of the peptide) and which are formed with inorganic acids such as, for example, hydrochloric or phosphoric acids, or with organic acids such as acetic, oxalic, tartaric, maleic, and the like. Salts formed with the free carboxyl groups can also be derived from inorganic bases such as, for example, sodium, potassium, ammonium, calcium, or ferric hydroides, and such organic bases as isopropylamine, trimethylamine, 2-ethylamino ethanol, histidine, procaine, and the like.

Vaccine or treatment compositions are administered in a manner compatible with the dosage formulation, and in such amount as will be prophylactically and/or therapeutically effective. The quantity to be administered depends on the subject to be treated, including, e.g., capacity of the subject's immune system to synthesize antibodies, and the degree of protection or treatment desired. Suitable dosage ranges are of the order of several hundred micrograms active ingredient per vaccination with a range from about 0.1 mg to 1000 mg, such as in the range from about 1 mg to 300 mg, and preferably in the range from about 10 mg to 50 mg. Suitable regiments for initial administration and booster shots are also variable but are typified by an initial administration followed by subsequent inoculations or other administrations. Precise amounts of active ingredient required to be administered depend on the judgment of the practitioner and may be peculiar to each subject. It will be apparent to those of skill in the art that the therapeutically effective amount of nucleic acid molecule or fusion polypeptides of this invention will depend, inter alia, upon the administration schedule, the unit dose of antigen administered, whether the nucleic acid molecule or fusion polypeptide is administered in combination with other therapeutic agents, the immune status and health of the recipient, and the therapeutic activity of the particular nucleic acid molecule or fusion polypeptide.

The compositions can be given in a single dose schedule or in a multiple dose schedule. A multiple dose schedule is one in which a primary course of vaccination may include, e.g., 1-10 separate doses, followed by other doses given at subsequent time intervals required to maintain and or reinforce the immune response, for example, at 1-4 months for a second dose, and if needed, a subsequent dose(s) after several months. Periodic boosters at intervals of 1-5 years, usually 3 years, are desirable to maintain the desired levels of protective immunity. The course of the immunization can be followed by in vitro proliferation assays of peripheral blood lymphocytes (PBLs) co-cultured with ESAT6 or ST-CF, and by measuring the levels of IFN-γ released from the primed lymphocytes. The assays may be performed using conventional labels, such as radionucleotides, enzymes, fluorescent labels and the like. These techniques are known to one skilled in the art and can be found in U.S. Pat. Nos. 3,791,932, 4,174,384 and 3,949,064, relevant portions incorporated by reference.

The modular rAb carrier and/or conjugated rAb carrier-(cohesion/dockerin and/or dockerin-cohesin)-antigen complex (rAb-DC/DC-antigen vaccine) may be provided in one or more "unit doses" depending on whether the nucleic acid vectors are used, the final purified proteins, or the final vaccine form is used. Unit dose is defined as containing a predetermined-quantity of the therapeutic composition calculated to produce the desired responses in association with its administration, i.e., the appropriate route and treatment regimen. The quantity to be administered, and the particular route and formulation, are within the skill of those in the clinical arts. The subject to be treated may also be evaluated, in particular, the state of the subject's immune system and the protection desired. A unit dose need not be administered as a single injection but may include continuous infusion over a set period of time. Unit dose of the present invention may conveniently may be described in terms of DNA/kg (or protein/Kg) body weight, with ranges between about 0.05, 0.10, 0.15, 0.20, 0.25, 0.5, 1, 10, 50, 100, 1,000 or more mg/DNA or protein/kg body weight are administered. Likewise the amount of rAb-DC/DC-antigen vaccine delivered can vary from about 0.2 to about 8.0 mg/kg body weight. Thus, in particular embodiments, 0.4 mg, 0.5 mg, 0.8 mg, 1.0 mg, 1.5 mg, 2.0 mg, 2.5 mg, 3.0 mg, 4.0 mg, 5.0 mg, 5.5 mg, 6.0 mg, 6.5 mg, 7.0 mg and 7.5 mg of the vaccine may be delivered to an individual in vivo. The dosage of rAb-DC/DC-antigen vaccine to be administered depends to a great extent on the weight and physical condition of the subject being treated as well as the route of administration and the frequency of treatment. A pharmaceutical composition that includes a naked polynucleotide prebound to a liposomal or viral delivery vector may be administered in amounts ranging from 1 µg to 1 mg polynucleotide to 1 µg to 100 mg protein. Thus, particular compositions may include between about 1 µg, 5 µg, 10 µg, 20 µg, 30 µg, 40 µg, 50 µg, 60 µg, 70 µg, 80 µg, 100 µg, 150 µg, 200 µg, 250 µg, 500 µg, 600 µg, 700 µg, 800 µg, 900 µg or 1,000 µg polynucleotide or protein that is bound independently to 1 µg, 5 µg, 10 µg, 20 µg, 3.0 µg, 40 µg 50 µg, 60 µg, 70 µg, 80 µg, 100 µg, 150 µg, 200 µg, 250 µg, 500 µg, 600 µg, 700 µg, 800 µg, 900 µg, 1 mg, 1.5 mg, 5 mg, 10 mg, 20 mg, 30 mg, 40 mg, 50 mg, 60 mg, 70 mg, 80 mg, 90 mg or 100 mg vector.

The present invention was tested in an in vitro cellular system that measures immune stimulation of human Flu-specific T cells by dendritic cells to which Flu antigen has been targeted. The results shown herein demonstrate the specific expansion of such antigen specific cells at doses of the antigen which are by themselves ineffective in this system.

The present invention may also be used to make a modular rAb carrier that is, e.g., a recombinant humanized mAb (directed to a specific human dendritic cell receptor) complexed with protective antigens from Ricin, Anthrax toxin, and Staphylococcus B enterotoxin. The potential market for this entity is vaccination of all military personnel and stored vaccine held in reserve to administer to large population centers in response to any biothreat related to these agents. The invention has broad application to the design of vaccines in general, both for human and animal use. Industries of interest include the pharmaceutical and biotechnology industries.

The present invention includes compositions and methods, including vaccines, that specifically target (deliver) antigens to antigen-presenting cells (APCs) for the purpose of eliciting potent and broad immune responses directed against the antigen. These compositions evoke protective or therapeutic immune responses against the agent (pathogen or cancer) from which the antigen was derived. In addition the invention creates agents that are directly, or in concert with other agents, therapeutic through their specific engagement of a receptor called DC-ASGPR that is expressed on antigen-presenting cells.

Dectin-1 (CLEC7A; G.D. Brown, Nat Rev Immunol 6 (2006), pp. 33-43.) was originally reported as a Dendritic Cell (DC)-specific molecule with a T cell costimulatory capacity (N. Kanazawa et al., J Biol Chem 278 (2003), pp. 32645-32652), but later its expression was found more strongly in monocytes, macrophages and neutrophils, and weakly in a subset of T cells, and, in humans, B cells and eosinophils. Most importantly, it was determined that Dectin-1 recognizes fungal β-glucan as the major nonopsonic receptor and is critical for the biological effects of β-glucan (G.D. Brown, Nat Rev Immunol 6 (2006), pp. 33-43.). Expression of Dectin-1 on macrophages is upregulated by IL-4 and IL-13, and downregulated by LPS as well as IL-10 and dexamethasone. Dectin-1 uniquely contains ITAM in its cytoplasmic region and actually works as an activating receptor in the absence of any adaptor molecule. The structure of its ITAM is also unique. Compared with the consensus sequence containing two repeats of YxxL/I (tyrosine-any-any-leucine or isoleucine) (S.J. van Vliet et al., Int Immunol 17 (2005), pp. 661-669, three amino acid residues are inserted between the first tyrosine and following leucine or isoleucine (YxxxL/I) in mouse or human Dectin-1, respectively. Actually, phosphorylation of only the second tyrosine was reportedly sufficient for the recruitment of Syk and various activatory effects of Dectin-1, such as induction of phagocytosis, production of reactive oxygen species (ROS) and cytokine production mediated by nuclear factor (NF)-κB activation. However, Syk was required for only some of these effects in a certain cell type (macrophage or DCs). In addition, some of these effects required cooperation with MyD88-mediated TLR signaling. Notably, the Dectin-1 signal in DCs also induced IL-10 production in a Syk-dependent manner. Interestingly, β-glucan is exposed on the yeast cell surface only under some specific conditions and loss of the Dectin-1 signal might explain why some fungi escape the host immune surveillance (S.E. Heinsbroek et al., Trends Immunol 26 (2005), pp. 352-354). In addition, the Dectin-1-mediated signal was reported to promote induction of autoimmune arthritis in the genetically predisposed (ZAP-70-mutated) SKG mouse (H. Yoshitomi et al., J Exp Med 201 (2005), pp. 949-960). Considering that Candida antigens have widely been used for the induction of various autoimmune disease models (E.C. Keystone et al., Arthritis Rheum 20 (1977), pp. 1396-1401), Dectin-1 may have a role in breakage of self-tolerance. Its possible association with regulatory T cells, similar to that of Dectin-2 (E.P. McGreal, M. Rosas, G.D. Brown, S. Zamze, S.Y. Wong and S. Gordon et al., Glycobiology 16 (2006), pp. 422-430.), however, remains to be elucidated.

DCs can cross-present protein antigens (Rock KL Immunol Rev. 2005 Oct;207:166-83). In vivo, DCs take up antigens by the means of a number of receptors and present antigenic peptides in both class I and II. In this context, DC lectins, as pattern recognition receptors, contribute to the efficient uptake of antigens as well as cross-presentation of antigens.

The present invention includes the development, characterization and use of anti-human Dectin-1 monoclonal antibodies (mAbs) and characterized their biological functions leading to discovery of likely therapeutic applications of anti-Dectin-1 mAbs and their surrogates. The invention disclosure reveals means of developing unique agents capable of activating cells bearing Dectin-1, as well as the effect of the resulting changes in cells receiving these signals regards action on other cells in the immune system. These effects [either alone, or in concert with other signals (i.e., co-stimulation)] are highly predictive of therapeutic outcomes for certain disease states or for augmenting protective outcomes in the context of vaccination.

### Materials and Methods

Antibodies and tetramers -Antibodies (Abs) for surface staining of DCs and B cells, including isotype control Abs, were purchased from BD Biosciences (CA). Abs for ELISA were purchased from Bethyl (TX). Anti-BLyS and anti-APRIL were from PeproTech (NJ). Tetramers, HLA-A*0201-GILGFVFTL (Flu M1) and HLA-A*0201-ELAGIGILTV (Mart-1), were purchased from Beckman Coulter (CA).

Cells and cultures - Monocytes (1x10⁶/ml) from normal donors were cultured in Cellgenics (France) media containing GM-CSF (100 ng/ml) and IL-4 (50 ng/ml) (R&D, CA). For day three and day six, DCs, the same amounts of cytokines were supplemented into the media on day one and day three, respectively. B cells were purified with a negative isolation kit (BD). CD4 and CD8 T cells were purified with magnetic beads coated with anti-CD4 or CD8 (Milteniy, CA). PBMCs were isolated from Buffy coats using Percoll™ gradients (GE Healthcare UK Ltd, Buckinghamshire, UK) by density gradient centrifugation. For DC activation, 1x10⁵ DCs were cultured in the mAb-coated 96-well plate for 16-18 h. mAbs (1-2 ug/well) in carbonate buffer, pH 9.4, were incubated for at least 3h at 37°C. Culture supernatants were harvested and cytokines / chemokines were measured by Luminex (Biorad, CA). For gene analysis, DCs were cultured in the plates coated with mAbs for 8 h. In some studies, soluble 50 ng/ml of CD40L (R&D, CA) or 50 nM CpG (InVivogen, CA) was added into the cultures. In the DCs and B cell co-cultures, 5x10³ DCs resuspended in RPMI 1640 with 10% FCS and antibiotics (Biosource, CA) were first cultured in the plates coated with mAbs for at least 6 h, and then 1x10⁵ purified autologous B cells labeled with CFSE (Molecular Probes, OR) were added. In some studies, DCs were pulsed with 5 moi (multiplicity of infection) of heat-inactivated influenza virus (A/PR/8 H1N1) for 2 h, and then mixed with B cells. For the DCs and T cell co-cultures, 5x10³ DCs were cultured with 1x10⁵ purified autologous CD8 T cells or mixed allogeneic T cells. Allogeneic T cells were pulsed with 1 uCi/well ³[H]-thymidine for the final 18h of incubation, and then cpm were measured by a gamma-counter (Wallac, MN). 5x10⁵ PBMCs /well were cultured in the plates coated with mAbs. The frequency of Mart-1 and Flu M1 specific CD8 T cells was measured by staining cells with anti-CD8 and tetramers on day ten and day seven of the cultures, respectively. 10 uM of Mart-1 peptide (ELAGIGILTV) and 20 nM of recombinant protein containing Mart-1 peptides (see below) were added to the DC and CD8 T cell cultures. 20 nM purified recombinant Flu M1 protein (see below) was add to the PBMC cultures.

Monoclonal antibodies - Mouse mAbs were generated by conventional technology. Briefly, six-week-old BALB/c mice were immunized i.p. with 20 µg of receptor ectodomain.hIgGFc fusion protein with Ribi adjuvant, then boosts with 20 µg antigen ten days and fifteen days later. After three months, the mice were boosted again three days prior to taking the spleens. Alternately, mice were injected in the footpad with 1-10 µg antigen in Ribi adjuvant every three to four days over a thirty to forty day period. Three to four days after a final boost, draining lymph nodes were harvested. B cells from spleen or lymph node cells were fused with SP2/O-Ag 14 cells. Hybridoma supernatants were screened to analyze Abs to the receptor ectodomain fusion protein compared to the fusion partner alone, or the receptor ectodomain fused to alkaline phosphatase (15). Positive wells were then screened in FACS using 293F cells transiently transfected with expression plasmids encoding full-length receptor cDNAs. Selected hybridomas were single cell cloned and expanded in CELLine flasks (Integra, CA). Hybridoma supernatants were mixed with an equal volume of 1.5 M glycine, 3 M NaCl, 1x PBS, pH 7.8 and tumbled with MabSelect resin. The resin was washed with binding buffer and eluted with 0.1 M glycine, pH 2.7. Following neutralization with 2 M Tris, mAbs were dialyzed versus PBS.

ELISA - Sandwich ELISA was performed to measure total IgM, IgG, and IgA as well as flu-specific immunoglobulins (Igs). Standard human serum (Bethyl) containing known amounts of Igs and human AB serum were used as standard for total Igs and flu-specific Igs, respectively. Flu specific Ab titers, units, in samples were defined as dilution factor of AB serum that shows an identical optical density. The amounts of BAFF and BLyS were measured by ELISA kits (Bender MedSystem, CA).

RNA purification and gene analysis - Total RNA extracted with RNeasy columns (Qiagen), and analyzed with the 2100 Bioanalyser (Agilent). Biotin-labeled cRNA targets were prepared using the Illumina totalprep labeling kit (Ambion) and hybridized to Sentrix Human6 BeadChips (46K transcripts). These microarrays consist of 50mer oligonucleotide probes attached to 3um beads which are lodged into microwells etched at the surface of a silicon wafer. After staining with Streptavidin-Cy3, the array surface is imaged using a sub-micron resolution scanner manufactured by Illumina (Beadstation 500X). A gene expression analysis software program, GeneSpring, Version 7.1 (Agilent), was used to perform data analysis.

Expression and purification of recombinant Flu M1 and MART-1 proteins - PCR was used to amplify the ORF of Influenza A/Puerto Rico/8/34/Mount Sinai (H1N1) M1 gene while incorporating an Nhe I site distal to the initiator codon and a Not I site distal to the stop codon. The digested fragment was cloned into pET28b(+) (Novagen), placing the M1 ORF in-frame with a His6 tag, thus encoding His.Flu M1 protein. A pET28b (+) derivative encoding an N-terminal 169 residue cohesin domain from *C*. *thermocellum* (unpublished) inserted between the Nco I and Nhe I sites expressed Coh.His. For expression of Cohesin-Flex-hMART-1-PeptideA-His, the sequence GACACCACCGAGGCCCGCCACCCCCACCCCCCCGTGACCACCCCCACCACCACCGA CCGGAAGGGCACCACCGCCGAGGAGCTGGCCGGCATCGGCATCCTGACCGTGATCC TGGGCGGCAAGCGGACCAACAACAGCACCCCCACCAAGGGCGAATTCTGCAGATAT CCATCACACTGGCGGCCG (SEQ ID NO.: 1)(encoding DTTEARHPHPPVTTPTTDRKGTTAEELAGIGILTVILGGKRTNNSTPTKGEFCRYPSHWR P (SEQ ID NO.: 2)- the shaded residues are the immuno-dominant HLA-A2-restricted peptide and the underlined residues surrounding the peptide are from MART-1) was inserted between the Nhe I and Xho I sites of the above vector. The proteins were expressed in *E*. *coli* strain BL21 (DE3) (Novagen) or T7 Express (NEB), grown in LB at 37°C with selection for kanamycin resistance (40 µg/ml) and shaking at 200 rounds/min to mid log phase growth when 120 mg/L IPTG was added. After three hours, the cells were harvested by centrifugation and stored at - 80°C. *E*. *coli* cells from each 1 L fermentation were resuspended in 30 ml ice-cold 50 mM Tris, 1 mM EDTA pH 8.0 (buffer B) with 0.1 ml of protease inhibitor Cocktail II (Calbiochem, CA). The cells were sonicated on ice 2x 5 min at setting 18 (Fisher Sonic Dismembrator 60) with a 5 min rest period and then spun at 17,000 r.p.m. (Sorvall SA-600) for 20 min at 4°C. For His.Flu M1 purification the 50 ml cell lysate supernatant fraction was passed through 5 ml Q Sepharose beads and 6.25 ml 160 mM Tris, 40 mM imidazole, 4 M NaCl pH 7.9 was added to the Q Sepharose flow through. This was loaded at 4 ml/min onto a 5 ml HiTrap chelating HP column charged with Ni++. The column-bound protein was washed with 20 mM NaPO₄, 300 mM NaCl pH 7.6 (buffer D) followed by another wash with 100 mM H₃COONa pH 4.0. Bound protein was eluted with 100 mM H₃COONa pH 4.0. The peak fractions were pooled and loaded at 4 ml/min onto a 5 ml HiTrap S column equilibrated with 100 mM H₃COONa pH 5.5, and washed with the equilibration buffer followed by elution with a gradient from 0 - 1 M NaCl in 50 mM NaPO₄ pH 5.5. Peak fractions eluting at about 500 mM NaCl were pooled. For Coh.Flu M1.His purification, cells from 2 L of culture were lysed as above. After centrifugation, 2.5 ml of Triton X114 was added to the supernatant with incubation on ice for 5 min. After further incubation at 25°C for 5 min, the supernatant was separated from the Triton X114 following centrifugation at 25°C. The extraction was repeated and the supernatant was passed through 5 ml of Q Sepharose beads and 6.25 ml 160 mM Tris, 40 mM imidazole, 4 M NaCl pH 7.9 was added to the Q Sepharose flow through. The protein was then purified by Ni⁺⁺ chelating chromatography as described above and eluted with 0-500 mM imidazole in buffer D.

Anti-Dectin-1 mAbs - The invention encompasses particular amino acid sequences shown below corresponding to anti-LOX-1 monoclonal antibodies that are desirable components (in the context of e.g., humanized recombinant antibodies) of therapeutic or protective products. The following are such sequences in the context of chimeric mouse V region (underlined) - human C region (bold) recombinant antibodies. These mouse V regions can be readily 'humanized, i.e., the LOX-1 combining regions grafted onto human V region framework sequences, by anyone well practiced in this art. Furthermore, the sequences can also be expressed in the context of fusion proteins that preserve antibody functionality, but add e.g., antigen, cytokine, or toxin for desired therapeutic applications.
rAB-pIRES2[manti-Dectin_1_**11B6**.4_**H**-V-hIgG4H-C]
rAB-pIRES2[manti-Dectin_1_**11B6**.4_**K**-V-hIgGK-C]
rAB-pIRES2[manti-Dectin_1_**15E2**.5_**H**-V-hIgG4H-C]
rAB-pIRES2[manti-Dectin_1_**15E2**.5_**K**-V-hIgGK-C]
rAB-pIRES2[mAnti-Dectin_1_**2D8**.2D4**H**-V-hIgG4H-C]
rAB-pIRES2[mAnti-Dectin_1_**2D8**.2D4**K**-V-hIgGK-C]

The V-region sequences and related sequences can be modified by those well versed in the art to, e.g., enhance affinity for Dectin-1 and/or integrated into human V-region framework sequences to be engineered into expression vectors to direct the expression of protein forms that can bind to Dectin-1 on antigen presenting cells. Furthermore, the other mAbs disclosed in the invention (or derived using similar methods and screens for the unique biology disclosed herein), can be via similar means (initially via PCR cloning and sequencing of mouse hybridoma V regions) be rendered into expression constructs encoding similar recombinant antibodies (rAbs). Such anti-Dectin-1 V regions can furthermore, by those well versed in the art, be 'humanized (i.e., mouse -specific combining sequences grafted onto human V region framework sequences) so as to minimize potential immune reactivity of the therapeutic rAb.

Engineered recombinant anti-Dectin-1 recombinant antibody - antigen fusion proteins (rAb.antigen) are efficacious prototype vaccines in vitro - Expression vectors can be constructed with diverse protein coding sequence e.g., fused in-frame to the H chain coding sequence. For example, antigens such as Influenza HA5, Influenza M1, HIV gag, or immuno-dominant peptides from cancer antigens, or cytokines, can be expressed subsequently as rAb.antigen or rAb.cytokine fusion proteins, which in the context of this invention, can have utility derived from using the anti-Dectin-1 V-region sequence to bring the antigen or cytokine (or toxin) directly to the surface of the antigen presenting cell bearing Dectin-1. This permits internalization of e.g., antigen - sometimes associated with activation of the receptor and ensuing initiation of therapeutic or protective action (e.g., via initiation of a potent immune response, or via killing of the targeted cell. An exemplative prototype vaccine based on this concept could use a H chain vector such as rAB-pIRES2[manti-Dectin_1_11B6.4_H-LV-hIgG4H-C-Flex-FluHA1-1-6xHis] which directs the synthesis of a H chain mouse mAb V region (underlined) -human IgG4 C region (bold)-Flu HA1-1 (italicized) fusion protein. Co-expressed with the analogous light chain expression plasmid (encoding sequence shown above), this produces a multifunctional recombinant antibody (rAb) that binds Dectin-1 and delivers Flu HA1-1 antigen to the cell surface for DC activation, antigen internalization, antigen processing, antigen presentation, and education and expansion of specific anti-Flu HA1-1 B and T cells. Similar H chain constructs with fusions to FluHA5-1, HIV Viralgag p24, and PSA (prostate-specific antigen) are also shown below.
rAB-pIRES2[manti-Dectin_1_11B6.4_H-LV-hIgG4H-C-Flex-FluHA1-1-6xHis]
rAB-pIRES2[manti-Dectin_1_11B6.4 H-LV-hIgG4H-C-Flex-FluHA5-1-6xHis]
rAB-pIRES2[mAnti-Dectin_1_2D8.2D4H-LV-hIgG4H-Viralgag]
rAB-pIRES2 [mAnti-Dectin_1-2D8.2D4H-V-hIgG4H-C-hPSA]

Similarly, rAB-pIRES2[manti-Dectin_1_11B6.4_H-LV-hIgG4H-C-Dockerin] shown below encodes an anti-Dectin-1 H chain fused to dockerin (sequence shown below). The dockerin allows for strong specific non-covalent interaction with cohesin-antigen (coh.antigen) fusion proteins - providing an alternate means of delivering antigen to DC and other antigen presenting cells.
rAB-pIRES2[manti-Dectin₋1₋1 IB6.4-H-LV-hlgG4H-C-Dockerin]

Methods relating to cloning and expression of recombinant antibodies (rAbs) and rAb.antigens cDNA cloning and expression of chimeric mouse/human mAbs - Total RNA was prepared from hybridoma cells (RNeasy kit, Qiagen) and used for cDNA synthesis and PCR (SMART RACE kit, BD Biosciences) using supplied 5' primers and gene specific 3' primers
(mIgGκ, 5'ggatggtgggaagatggatacagttggtgcagcatc3' (SEQ ID NO.: 14);
mIgGλ, 5'ctaggaacagtcagcacgggacaaactcttctccacagtgtgaccttc3'(SEQ ID NO.: 15);
mIgG1, 5'gtcactggctcagggaaatagcccttgaccaggcatc3' (SEQ ID NO.: 16);
mIgG2a, 5'ccaggcatcctagagtcaccgaggagccagt3'(SEQ ID NO.: 17); and
mIgG2b, 5'ggtgctggaggggacagtcactgagctgctcatagtgt3'(SEQ ID NO.: 18)).

PCR products were cloned (pCR2.1 TA kit, Invitrogen) and characterized by DNA sequencing. Using the derived sequences for the mouse H and L chain V-region cDNAs, specific primers were used to PCR amplify the signal peptide and V-regions while incorporating flanking restriction sites for cloning into expression vectors encoding downstream human IgGκ or IgG4H regions. The vector for expression of chimeric mVκ-hIgκ was built by amplifying residues 401-731 (gi|63101937|) flanked by *Xho* I and Not I sites and inserting this into the Xho I - Not I interval of pIRES2-DsRed2 (BD Biosciences). PCR was used to amplify the mAb Vk region from the initiator codon, appending a *Nhe* I or *Spe* I site then CACC, to the region encoding (e.g., residue 126 of gi|76779294|), appending a *Xho* I site. The PCR fragment was then cloned into the *Nhe* I - *Not* I interval of the above vector. The vector for chimeric mVκ-hIgK using the mSLAM leader was built by inserting the sequence 5'ctagttgctggctaatggaccccaaaggctccctttcctggagaatacttctgtttctctccctggcttttgagttgtcgtacggattaattaag ggcccactcgag3' (SEQ ID NO.: 19) into the *Nhe* I - *Xho* I interval of the above vector. PCR was used to amplify the interval between the predicted mature N-terminal codon (defined using the SignalP 3.0 Server) (16) and the end of the mVκ region (as defined above) while appending 5'tcgtacgga3'. The fragment digested with *Bsi* WI and *Xho* I was inserted into the corresponding sites of the above vector. The control hIgκ sequence corresponds to gi|49257887| residues 26-85 and gi|21669402| residues 67-709. The control hIgG4H vector corresponds to residues 12-1473 of gi|19684072| with S229P and L236E substitutions, which stabilize a disulphide bond and abrogate residual FcR binding (27), inserted between the pIRES2-DsRed2 vector Bgl II and Not I sites while adding the sequence 5'gctagctgattaattaa3' (SEQ ID NO.: 20) instead of the stop codon. PCR was used to amplify the mAb VH region from the initiator codon, appending CACC then a *Bgl* II site, to the region encoding residue 473 of gi|19684072|. The PCR fragment was then cloned into the *Bg*l II - *Apa* I interval of the above vector. The vector for chimeric mVH-hIgG4 sequence using the mSLAM leader was built by inserting the sequence 5'ctagttgctggctaatggaccccaaaggctccctttcctggagaatacttctgtttctctccctggcttttgagttgtcgtacggattaattaag ggccc3' (SEQ ID NO.: 22) into the *Nhe* I - *Apa* I interval of the above vector. PCR was used to amplify the interval between the predicted mature N-terminal codon and the end of the mVH region while appending 5'tcgtacgga3'. The fragment digested with Bsi WI and Apa I was inserted into the corresponding sites of the above vector. Appendix 2 details the nucleotide sequences of the various mVκ and mVH regions used in this study.

Various antigen coding sequences flanked by a proximal *Nhe* I site and a distal *Not* I site following the stop codon were inserted into the *Nhe* I - Pac I - *Not* I interval of the H chain vectors. Flu HA1-1 was encoded by Influenza A virus (A/Puerto Rico/8/34(H1N1)) hemagglutinin gi|21693168| residues 82-1025 (with a C982T change) with proximal 5'gctagcgatacaacagaacctgcaacacctacaacacctgtaacaa3' (SEQ ID NO.: 23) sequence (a *Nhe* I site followed by sequence encoding cipA cohesin-cohesin linker residues) and distal 5'caccatcaccatcaccattgagcggccgc3' (SEQ ID NO.: 24) sequence (encoding His6, a stop codon, and a Not I site). Flu HA5-1 was encoded by gi|50296052| Influenza A virus (A/Viet Nam/1203/2004(H5N1)) hemagglutinin residues 49-990 bound by the same sequences as Flu HA1-1. Doc was encoded by gi|40671| celD residues 1923-2150 with proximal *Nhe* I and distal Not I sites. PSA was encoded by gi|34784812| prostate specific antigen residues 101-832 with proximal sequence 5'gctagcgatacaacagaacctgcaacacctacaacacctgtaacaacaccgacaacaacacttctagcgc3' (SEQ ID NO.: 25) (Nhe I site and cipA spacer) and a distal *Not* I site. Flu M1-PEP was encoded by 5'gctagccccattctgagccccctgaccaaaggcattctgggctttgtgtttaccctgaccgtgcccagcgaacgcaagggtatacttggat tcgttttcacacttacttaagcggccgc3' (SEQ ID NO.: 26). This and all other peptide-encoding sequences were created via mixtures of complimentary synthetic DNA fragments with ends compatible for cloning into *Nhe* I and *Not* I-restricted H chain vectors, or *Nhe* I - *Xho* I-restricted Coh.His vector. Preferred human codons were always used, except where restriction sites needed to be incorporated or in CipA spacer sequences.

Production levels of rAb expression constructs were tested in 5 ml transient transfections using ~2.5 µg each of the L chain and H chain construct and the protocol described above. Supernatants were analyzed by anti-hIgG ELISA (AffiniPure Goat anti-human IgG (H+L), Jackson ImmunoResearch). In tests of this protocol, production of secreted rAb was independent of H chain and L chain vectors concentration over a ~fold range of each DNA concentration (i.e., the system was DNA saturated).

Novel anti-Dectin-1 monoclonal antibodies (mAbs) were developed and used to uncover previously unknown biology associated with this cell surface receptor that is found on antigen-presenting cells. This novel biology is highly predictive of the application of anti-Dectin-1 agents that activate this receptor for diverse therapeutic and protective applications. Data presented below strongly support the initial predictions and demonstrated the pathway to reducing the discoveries herein to clinical application.

Development of high affinity monoclonal antibodies against human Dectin-1: Receptor ectodomain.hIgG (human IgG1Fc) and AP (human placental alkaline phosphatase) fusion proteins were produced for immunization of mice and screening of mAbs, respectively. An expression construct for DCIR ectodomain.IgG was described previously (15) and used the mouse SLAM (mSLAM) signal peptide to direct secretion (16). An expression vector for DCIR ectodomain.AP was also generated using PCR to amplify AP resides 133-1581 (gb|BC009647|) while adding a proximal in-frame *Xho* I site and a distal TGA stop codon and Not I site. This *Xho* I - *Not* I fragment replaced the IgG coding sequence in the above DCIR ectodomain.IgG vector. Dectin-1 ectodomain constructs in the same Ig and AP vector series contained inserts encoding (bp 397-603, gi|88999591|. Dectin-1 fusion proteins were produced using the FreeStyle™ 293 Expression System (Invitrogen) according to the manufacturer's protocol (1 mg total plasmid DNA with 1.3 ml 293 Fectin reagent /L of transfection). For rAb production, equal amounts of vector encoding the H and L chain were co-transfected. Transfected cells are cultured for 3 days, the culture supernatant was harvested and fresh media added with continued incubation for two days. The pooled supernatants were clarified by filtration. Receptor ectodomain.hIgG was purified by HiTrap protein A affinity chromatography with elution by 0.1 M glycine pH 2.7 and then dialyzed versus PBS. rAbs (recombinant antibodies described later)were purified similarly, by using HiTrap MabSelect™ columns. Mouse mAbs were generated by conventional cell fusion technology. Briefly, 6-week-old BALB/c mice were immunized intraperitonealy with 20 µg of receptor ectodomain.hIgGFc fusion protein with Ribi adjuvant, then boosts with 20 µg antigen 10 days and 15 days later. After 3 months, the mice were boosted again three days prior to taking the spleens. Alternately, mice were injected in the footpad with 1-10 µg antigen in Ribi adjuvant every 3-4 days over a 30-40 day period. 3-4 days after a final boost, draining lymph nodes were harvested. B cells from spleen or lymph node cells were fused with SP2/O-Ag 14 cells (17) using conventional techniques. ELISA was used to screen hybridoma supernatants against the receptor ectodomain fusion protein compared to the fusion partner alone, or versus the receptor ectodomain fused to AP (15). Positive wells were then screened in FACS using 293F cells transiently transfected with expression plasmids encoding full-length receptor cDNAs. Selected hybridomas were single cell cloned, adapted to serum-free media, and expanded in CELLine flasks (Intergra). Hybridoma supernatants were mixed with an equal volume of 1.5 M glycine, 3 M NaCl, 1x PBS, pH 7.8 and tumbled with MabSelect resin. The resin was washed with binding buffer and eluted with 0.1 M glycine, pH 2.7. Following neutralization with 2 M Tris, mAbs were dialyzed versus PBS.

Characterization of purified anti-Dectin-1 monoclonal antibodies by direct ELISA. The relative affinities of several anti-Dectin-1 mAbs by ELISA (i.e., Dectin-1.Ig protein is immobilized on the microtiter plate surface and the antibodies are tested in a dose titration series for their ability to bind to Dectin-1.Ig (as detected by an anti-mouse IgG-HRP conjugate reagent). The panels are (left) mAb reactivity to Dectin-1.Ig protein; and (right) mAb reactivity to hIgGFc protein. The panels show that the anti-Dectin-1 mAbs react specifically to the Dectin-1. Variations in binding were noted for the various antibodies.

Both in vivo DCs and in vitro-cultured DCs express Dectin-1: Figures 1A and 1B show the expression levels of Dectin-1 on PBMCs from normal donors. As shown in Fig. 1A below, antigen presenting cells, including CD11c+ DCs, CD 14+ monocytes, and CD19+B cells express Dectin-1. CD3+ T cells do not express detectable surface Dectin-1. Expression levels of Dectin-11 on in vitro-cultured DCs, IFNDCs and IL-4DCs, are shown in Fig. 1b. Both IL-4 and IFNDCs were stained with all three anti-Dectin-1 made in this study.

Both in vivo and in vitro-cultured DCs express Dectin-1. Figure 1A. PBMCs from normal donors were stained with anti-CD 11c, CD 14, CD 19, and CD3 with anti-Dectin-1 mAb. Cells stained with individual antibodies were gated to measure the expression levels of Dectin-1. Figure 1B. Monocytes from normal donors were cultured in the presence of GM-CSF with IL-4 (IL-4DCs) or IFNa (IFNDCs), and cells were stained with anti-Dectin-1 mAb. Cells were also stained with control antibodies and goat anti-mouse antibody labeled with FITC.

Expression levels of Dectin-1 on DCs are modulated with TLR ligands and cytokines: The expression levels of DC surface molecules can be modulated with various stimulators. To see whether Dectin-1 expression is controlled by the activation of DCs, IL-4DCs were stimulated with TLR ligands and cytokines. Data in Fig. 2 show that TLR4 ligand (LPS from E. coli) reduced the expression levels of Dectin-1, but IL-10 slightly enhanced Dectin-1 expression on IL-4DCs. In a repeat study, IL-15 slightly downregulated Dectin-1. Other stimulators, including IFNa and TLR3 ligand did not alter the expression levels of Dectin-1 on IL-4DCs.

Figure 2. In vitro-cultured IL-4DCs were cultured in the presence of CD40L (50 ng/ml), TLR2 ligand (100 ng/ml), TLR3 ligand (100 nM), TLR4 ligand (20 ng/ml), IL-10 (20 ng/ml), IL-15 (100 ng/ml), IFNa (500 U/ml), and IL-4 (50 ng/ml) for 24h. Cells were stained with anti-Dectin-1 or control mAbs. Dotted lines indicate cells stained with isotype control antibody. Gray and opened histograms represent cells cultured in the media without any stimulator and with stimulators, respectively.

Signaling through Dectin-1 stimulates DCs to produce cytokines and chemokines: Dendritic cells are the primary immune cells that determine the results of immune responses, either induction or tolerance, depending on their activation (18). The role of Dectin-1 in human DC activation has not been thoroughly studied. We generated 8 different hybridoma clones that produce mouse anti-hDectin-1 mAbs, and we tested whether individual mAbs activate DCs by measuring cytokines and chemokines secreted from DCs. Data in Fig. 3 show that especially PAB47 and PAB49 could activate DCs to produce significant amounts of IL-6, IL-8, IL-10, IL-12p40, IP-10, and MIP-1a. PAB187 is an anti-CD40 mAb that is known to activate DCs. PAB85 is a control antibody.

Figure 3 shows that anti-Dectin-1 mAbs activate DCs. 1x10e5/200 ul IFNDCs were cultured in the plates coated with different clones of mAbs for 18 h. A. Culture supernatants were analyzed to measure cytokines and chemokines by Luminex.

Figure 4 shows that signaling through Dectin-1 can result in the activation of both IL-4DCs and IFNDCs. For IFNDCs, anti-Dectin-1 mAbs stimulate DCs to express significantly increased levels of CD86, CD83, CD80, and HLA-DR (Fig. 4A). Anti-Dectin-1 mAb also activated IL-4DCs to express increased levels of CD86, CD80, and HLA-DR (Fig. 4B).

Signaling through Dectin-1 augments signaling through TLRs: Signals through Dectin-1 can synergize with signals through TLRs for enhanced activation of DCs (Fig. 5). It has been known that Dectin-1 synergizes with TLR2. In this study, however, we show that synergy between Dectin-1 and TLR4 is stronger than synergy between Dectin-1 and TLR2 for the activation of DCs. This synergy, Dectin-1 and TLR4, resulted in dramatically increased production of IL-10, IL-1b, TNFα, and IL-12p40 from DCs. We have also stained cells with anti-costimulatory molecules, including CD86, CD80, CD83 and CD40; this synergy did not result in increased expression of costimulatory molecules tested.

Synergy between Dectin-1 and TLR4 resulted in the enhanced antigen specific CD8 T cell responses: To test whether the synergy between Dectin-1 and TLR-mediated signaling results in enhanced immune responses, the capacity of DC to prime Mart-1 specific CD8 T cells was tested (Fig. 6). As shown in Fig. 6A, synergy between Dectin-1 and TLR4L resulted in more than 5 fold increased number of Mart-1 specific CD8 T cell responses. We could also see the synergy between Dectin-1 and TLR2 resulted in enhanced Mart-1 specific CD8 T cell priming. Data in Fig. 6 will be useful for future vaccine development.

IL-10 produced from DCs stimulated through Dectin-1 contributes to the enhanced Mart-1 specific CD8 T cell priming: As shown in Fig. 3 and Fig. 5, signaling through Dectin-1 resulted in significant amounts of IL-10, an inhibitory cytokine for CD8 T cell responses, from DCs. The amount of IL-10 were significantly increased when Dectin-1 synergize with TLR ligands. Regardless of the IL-10 produced by DCs, however, DCs stimulated with either anti-Dectin-1 alone or anti-Dectin-1 and TLR ligands resulted in the enhanced priming of Mart-1 specific CD8 T cells (Fig. 6). Therefore, it is likely that IL-10 during priming may contribute to antigen specific CD8 T cell priming. To test this hypothesis, we added anti-IL-10 antibody in the culture, and data in Fig. 7 indicate that IL-10 from DCs stimulated with anti-Dectin-1 and TLR ligands enhance Mart-1 specific CD8 T cell priming. Similar result was observed when DCs were stimulated with Zymosan. When same DCs were stimulated with Zymosan, DCs produced significant amount of IL-10 (more than 1000 pg/ml from 1x10e5 DCs) (data not shown). Since DCs stimulated with anti-Dectin-1 mAbs produce significant amounts of IL-10, this IL-10 mediated enhanced priming of antigen specific CD8 T cells will be useful for future vaccine development.

DCs activated with anti-Dectin-1 express increased levels of IL-15 and 4-1BBL: To further analyze the role of anti-Dectin-1 in DC activation, IFN DCs were stimulated with anti-Dectin-1, and then cells were stained with anti-IL-15 and 4-1BBL. Data in Fig. 8 show that signaling through Dectin-1 induces increased expression of both IL-15 and 4-1BBL that may contribute to enhanced humoral and cellular immune responses. IL-15 is known to playing an important role in CD8 T cell responses and B cell proliferation and antibody production. 4-1-BBL is a costimulatory molecule that contributes to DC-mediated CD8 T cell responses - human 4-1BBL signals through both CD137/4-1BB and itself. Its cytoplasmic tail participates in reverse signaling that induces apoptosis in T cells and cytokine secretion (IL-6; TNF-α) by monocytes.4-1BBL binding to CD137/4-1BB produces a number of effects and plays a key role in the T cell recall response. It maintains T cell numbers at the end of a primary response, and induces CD4+ and CD8+ T cells to proliferate and secrete cytokines such as IL-2 and IFN-γ in CD4+ cells, and IFN-γ in CD8+ cells.

DCs stimulated through Dectin-1 induce potent humoral immune responses:-DCs play an important role in humoral immune responses by providing signals for both T-dependent and T-independent B cell responses (19-22) and by transferring antigens to B cells (23, 24). In addition to DCs, signaling through TLR9 as a third signal is necessary for efficient B cell responses (25, 26). Dectin-1 can affect DCs-mediated humoral immune responses in the presence of TLR9 ligand, CpG. Six day IL-4 DCs were stimulated with anti- Dectin-1 mAb, and then purified B cells were co-cultured in the presence of CD40L or CpG. As shown in Fig. 9a and 9b, DCs activated with anti-Dectin-1 mAb result in the enhanced B cell proliferation (seen via CFSE dilution) and plasma cell differentiation (increase in CD38⁺), compared to DCs stimulated with control mAb.

The amounts of total immunoglobulins (Igs) produced were measured by ELISA (Fig. 9a and b). Consistent with the B cell proliferation and plasma cell differentiation, B cells cultured with anti-Dectin-1 mAb-stimulated DCs result in significantly increased production of total immunoglobulins in presence of CD40L or CpG.

The present invention may be used for the therapeutic application of agents that activate specifically through Dectin-1, e.g., as adjuvant in vaccination, or as immune system stimulants for immune compromised individuals. Also, the discovery predicts that blocking natural or abnormally regulated activation via Dectin-1 can have applications (e.g., for evoking tolerance in a transplantation setting, or ameliorating autoimmune diseases).

Anti-Dectin-1 mAbs activate B cells: CD19+ B cells express Dectin-1 (Fig. 1) and this predicts a possible role for Dectin-1 expressed on B cells. Data in Fig. 10A show that triggering Dectin-1 on B cells results in the enhanced B cell proliferation and plasma cell differentiation in the presence of CpG. Consistently, Fig. 10B shows that B cells activated with anti-Dectin-1 mAb secret increased amounts of total IgM, but not IgG and IgA.

The above discovery of direct effects on B cells through engagement of Dectin-1 reinforces the scope of therapeutic application outlined above for agents acting through or against Dectin-1.

Role of Dectin-1 in T cell responses: DCs stimulated through Dectin-1 express enhanced levels of co-stimulatory molecules and produce increased amounts of cytokines and chemokines (Fig. 3 and 4), suggesting that Dectin-1 contributes to enhanced cellular immune responses as well as humoral immune responses. This was tested by measuring antigen specific CD8 T cell responses (Fig. 11). Data in Fig. 11 show that DCs activated through Dectin-1 result in enhanced memory CD8 T cell responses specific to Flu M1 protein (Fig. 11a). More importantly, signaling through Dectin-1 permits DCs to prime and cross-prime Mart-1 peptides to CD8 T cells (Fig. 11b and c). This indicates that Dectin-1 plays an important role in enhancing DC function, resulting in improved priming and cross-priming of antigens to CD8 T cells. Therefore, anti-Dectin-1 mAb and antigen fusion proteins will induce robust antigen specific CD8 T cell responses, suggesting that reagents composed of anti-Dectin-1 mAbs will be useful for developing vaccine against cancers and infections.

Soluble form of anti-Dectin-1 mAbs activate DCs to result in enhanced priming of Mart-1 specific CD8 T cells from normal donors. We have shown that soluble anti-Dectin-1 mAbs can activate DCs, and therefore we have tested whether DCs stimulated with soluble anti-Dectin-1 could enhance antigen specific CD8 T cell responses. Data in Fig. 12 show that DCs activated with soluble anti-Dectin-1 resulted in the enhanced priming of Mart-1 specific CD8 T cells from normal donors. Soluble form of anti-CD40 made in this study also resulted in the enhanced CD8 T cell priming.

Human tonsil B cells divided into two groups based on Dectin-1 expression: Cells from human tonsils were stained with anti-Dectin-1 and other antibodies. As shown in Fig. 13, CD3+ T cells do not express Dectin-1 (left panel). However, there were two groups of CD19+ B cells; one does not express Dectin-1 and the other group of CD19+B cells expresses Dectin-1 similarly to CD19+B cells from PBMC from normal donors. This may likely have implications for developing new anti-Dectin-1-based therapeutic reagents for cancers and vaccines.

Cells in human skins and lymph nodes express Dectin-1: Cells expressing Dectin-1 are detectable in human skins and Lymph nodes by IHC. Tissue sections were stained with the nuclear stain DAPI (Blue) and Anti-Dectin-1 (Red). Fig. 14 shows that both human skin and lymph nodes contain cells express significant levels of Dectin-1. This is an important observation since it reveals that cells in vivo express Dectin-1- in exactly the location (skin) that would be a preferred site for administration of e.g., anti-Dectin-1-targeted antigen.

In vivo DCs in non-human primate express Dectin-1- Blood DCs in non-human primates (Cynomolgus), monkey PBMC were stained with anti-Dectin-1 mAbs and antibodies to other cellular markers, CD3, CD14, CD11c, CD27, CD56, and CD16. Data in Fig. 15 show that both CD 14 and CD11c+ cells were stained with anti-human Dectin-1 mAbs. Unlike CD14+ and CD11c+ cells, CD3+, CD16+, CD27+, and CD56+ cells did not express Dectin-1 (data not shown). This data demonstrates the cross-reactivity of certain anti-human Dectin-1-1 mAbs against monkey antigen presenting cells. This greatly facilitates reducing the inventions described herein to practice, since monkey can be used as a valid model for both efficacy and safely studies of envisioned human therapies.

The discoveries shown above both enhance and broaden the scope of therapeutic application for agents acting though Dectin-1. Dectin-1 activation of DCs influences neighboring T cells directing them to increase proliferation. Furthermore, when antigen is present during this interaction, Dectin-1 activated DCs result in enhance expansion of antigen-specific T cells. This shows that Dectin-1 activation, e.g., in a vaccine setting, can direct selective expansion of antigen-specific naïve T cells - this together with the above direct and indirect effects of Dectin-1 activation on B cells, clearly predicts the expansion of antigen-specific B cells, and therefore production of antigen-specific antibody.

It is contemplated that any embodiment discussed in this specification can be implemented with respect to any method, kit, reagent, or composition of the invention, and vice versa. Furthermore, compositions of the invention can be used to achieve methods of the invention.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof' as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein.

### References

1. Figdor, C. G., Y. van Kooyk, and G. J. Adema. 2002. C-type lectin receptors on dendritic cells and Langerhans cells. Nat Rev Immunol 2:77-84.
2. Pyz, E., A. S. Marshall, S. Gordon, and G. D. Brown. 2006. C-type lectin-like receptors on myeloid cells. Ann Med 38:242-251.
3. Brown, G. D. 2006. Dectin-1: a signalling non-TLR pattern-recognition receptor. Nat Rev Immunol 6:33-43.
4. Geijtenbeek, T. B., D. J. Krooshoop, D. A. Bleijs, S. J. van Vliet, G. C. van Duijnhoven, V. Grabovsky, R. Alon, C. G. Figdor, and Y. van Kooyk. 2000. DC-SIGN-ICAM-2 interaction mediates dendritic cell trafficking. Nat Immunol 1:353-357.
5. Geijtenbeek, T. B., R. Torensma, S. J. van Vliet, G. C. van Duijnhoven, G. J. Adema, Y. van Kooyk, and C. G. Figdor. 2000. Identification of DC-SIGN, a novel dendritic cell-specific ICAM-3 receptor that supports primary immune responses. Cell 100:575-585.
6. d'Ostiani, C. F., G. Del Sero, A. Bacci, C. Montagnoli, A. Spreca, A. Mencacci, P. Ricciardi-Castagnoli, and L. Romani. 2000. Dendritic cells discriminate between yeasts and hyphae of the fungus Candida albicans. Implications for initiation of T helper cell immunity in vitro and in vivo. J Exp Med 191:1661-1674.
7. Fradin, C., D. Poulain, and T. Jouault. 2000. beta-1,2-linked oligomannosides from Candida albicans bind to a 32-kilodalton macrophage membrane protein homologous to the mammalian lectin galectin-3. Infect Immun 68:4391-4398.
8. Cambi, A., K. Gijzen, J. M. de Vries, R. Torensma, B. Joosten, G. J. Adema, M. G. Netea, B. J. Kullberg, L. Romani, and C. G. Figdor. 2003. The C-type lectin DC-SIGN (CD209) is an antigen-uptake receptor for Candida albicans on dendritic cells. Eur J Immunol 33:532-538.
9. Netea, M. G., J. W. Meer, I. Verschueren, and B. J. Kullberg. 2002. CD40/CD40 ligand interactions in the host defense against disseminated Candida albicans infection: the role of macrophage-derived nitric oxide. Eur J Immunol 32:1455-1463.
10. Lee, S. J., S. Evers, D. Roeder, A. F. Parlow, J. Risteli, L. Risteli, Y. C. Lee, T. Feizi, H. Langen, and M. C. Nussenzweig. 2002. Mannose receptor-mediated regulation of serum glycoprotein homeostasis. Science 295:1898-1901.
11. Maeda, N., J. Nigou, J. L. Herrmann, M. Jackson, A. Amara, P. H. Lagrange, G. Puzo, B. Gicquel, and O. Neyrolles. 2003. The cell surface receptor DC-SIGN discriminates between Mycobacterium species through selective recognition of the mannose caps on lipoarabinomannan. J Biol Chem 278:5513-5516.
12. Tailleux, L., O. Schwartz, J. L. Herrmann, E. Pivert, M. Jackson, A. Amara, L. Legres, D. Dreher, L. P. Nicod, J. C. Gluckman, P. H. Lagrange, B. Gicquel, and O. Neyrolles. 2003. DC-SIGN is the major Mycobacterium tuberculosis receptor on human dendritic cells. J Exp Med 197:121-127.
13. Geijtenbeek, T. B., S. J. Van Vliet, E. A. Koppel, M. Sanchez-Hernandez, C. M. Vandenbroucke-Grauls, B. Appelmelk, and Y. Van Kooyk. 2003. Mycobacteria target DC-SIGN to suppress dendritic cell function. J Exp Med 197:7-17.
14. Cooper, A. M., A. Kipnis, J. Turner, J. Magram, J. Ferrante, and I. M. Orme. 2002. Mice lacking bioactive IL-12 can generate protective, antigen-specific cellular responses to mycobacterial infection only if the IL-12 p40 subunit is present. J Immunol 168:1322-1327.
15. Bates, E. E., N. Fournier, E. Garcia, J. Valladeau, I. Durand, J. J. Pin, S. M. Zurawski, S. Patel, J. S. Abrams, S. Lebecque, P. Garrone, and S. Saeland. 1999. APCs express DCIR, a novel C-type lectin surface receptor containing an immunoreceptor tyrosine-based inhibitory motif. J Immunol 163:1973-1983.
16. Bendtsen, J. D., H. Nielsen, G. von Heijne, and S. Brunak. 2004. Improved prediction of signal peptides: SignalP 3.0. J Mol Biol 340:783-795.
17. Shulman, M., C. D. Wilde, G. Kohler, M. J. Shulman, N. S. Rees, D. Atefi, J. T. Homey, S. B. Eaton, W. Whaley, J. T. Galambos, H. Hengartner, L. R. Shapiro, and L. Zemek. 1978. A better cell line for making hybridomas secreting specific antibodies. Nature 276:269-270.
18. Banchereau, J., F. Briere, C. Caux, J. Davoust, S. Lebecque, Y. J. Liu, B. Pulendran, and K. Palucka. 2000. Immunobiology of dendritic cells. Annu Rev Immunol 18:767-811.
19. Wykes, M., and G. MacPherson. 2000. Dendritic cell-B-cell interaction: dendritic cells provide B cells with CD40-independent proliferation signals and CD40-dependent survival signals. Immunology 100:1-3.
20. Balazs, M., F. Martin, T. Zhou, and J. Kearney. 2002. Blood dendritic cells interact with splenic marginal zone B cells to initiate T-independent immune responses. Immunity 17:341-352.
21. Kikuchi, T., S. Worgall, R. Singh, M. A. Moore, and R. G. Crystal. 2000. Dendritic cells genetically modified to express CD40 ligand and pulsed with antigen can initiate antigen-specific humoral immunity independent of CD4+ T cells. Nat Med 6:1154-1159.
22. Dubois, B., J. M. Bridon, J. Fayette, C. Barthelemy, J. Banchereau, C. Caux, and F. Briere. 1999. Dendritic cells directly modulate B cell growth and differentiation. J Leukoc Biol 66:224-230.
23. Qi, H., J. G. Egen, A. Y. Huang, and R. N. Germain. 2006. Extrafollicular activation of lymph node B cells by antigen-bearing dendritic cells. Science 312:1672-1676.
24. Bergtold, A., D. D. Desai, A. Gavhane, and R. Clynes. 2005. Cell surface recycling of internalized antigen permits dendritic cell priming of B cells. Immunity 23:503-514.
25. Ruprecht, C. R., and A. Lanzavecchia. 2006. Toll-like receptor stimulation as a third signal required for activation of human naive B cells. Eur J Immunol 36:810-816.
26. Bernasconi, N. L., E. Traggiai, and A. Lanzavecchia. 2002. Maintenance of serological memory by polyclonal activation of human memory B cells. Science 298:2199-2202.
27. Reddy, M. P., C. A. Kinney, M. A. Chaikin, A. Payne, J. Fishman-Lobell, P. Tsui, P. R. Dal Monte, M. L. Doyle, M. R. Brigham-Burke, D. Anderson, M. Reff, R. Newman, N. Hanna, R. W. Sweet, and A. Truneh. 2000. Elimination of Fc receptor-dependent effector functions of a modified IgG4 monoclonal antibody to human CD4. J Immunol 164:1925-1933.

## Claims

1. A method for increasing the effectiveness of antigen presentation by a Dectin-1-expressing dendritic cell comprising contacting the dendritic cell with an anti-Dectin-1-specific antibody or fragment thereof, wherein the anti-Dectin-1-specific antibody or fragment thereof is bound to a first half of a Cohesin/Dockerin pair and wherein the anti-Dectin-1-specific antibody or fragment thereof is capable of activating the dendritic cell, wherein the dendritic cell is activated.

2. The method of claim 1 further comprising activating the dendritic cell with GM-CSF, Interferon alpha, or IL-4, or combinations thereof, wherein contact with the Dectin-1-specific antibody or fragment thereof increases the surface expression of CD83, CD86, CD80, or HLA DR on the dendritic cell.

3. The method of claims 1 or 2, further comprising co-activating the dendritic cell through a TLR4 receptor, wherein the cells increase cytokine and chemokine production, or secretion of IL-10, IL-1b, TNFα, IL-12p40 and combinations thereof.

4. The method of claim 3, wherein co-activating through the TLR4 receptor employs at least one of a TLR4 ligand, an anti-TLR4 antibody or fragments thereof, an anti-TLR4-anti-Dectin-1 hybrid antibody or fragment thereof, an anti-TLR4-anti-Dectin-11 ligand conjugate.

5. The method of any one of claims 1 to 4, wherein the first half of the Cohesin/Dockerin pair is associated with a second half of a Cohesin/Dockerin pair that is bound to an antigen or a cytokine or a combination of cytokine and antigen.

6. The method of any one of claims 1 to 5,
further comprising contacting the dendritic cell with monocytes, peripheral blood mononuclear cells, B cells, or combinations thereof.

7. A vaccine comprising a dendritic cell activated with a Dectin-1-specific antibody or fragment thereof, preferably comprising at least one of SEQ ID NOS. 1-13, wherein the anti-Dectin-1-specific antibody or fragment thereof is bound to a first half of a Cohesin/Dockerin pair associated with a second half of a Cohesin/Dockerin pair that is bound to an antigen or a cytokine or a combination of cytokine and antigen.

## Patentansprüche

1. Verfahren zur Erhöhung der Wirksamkeit der Antigenpräsentation durch eine Dectin-1 exprimierende dendritische Zelle, umfassend das in Kontakt bringen der dendritischen Zelle mit einem Anti-Dectin-1-spezifischen Antikörper oder einem Fragment davon, wobei der Anti-Dectin-1-spezifische Antikörper oder das Fragment davon an eine erste Hälfte eines Cohesin/Dockerin-Paars gebunden ist und wobei der Anti-Dectin-1-spezifische Antikörper oder das Fragment davon dazu in der Lage ist, die dendritische Zelle zu aktiveren, wobei die dendritische Zelle aktiviert wird.

2. Verfahren nach Anspruch 1, des Weiteren umfassend das Aktivieren der dendritischen Zelle mit GM-CSF, Interferon alpha oder IL-4 oder Kombinationen aus diesen, wobei durch den Kontakt mit dem Dectin-1-spezifischen Antikörper oder dem Fragment davon die Oberflächenexpression von CD83, CD86, CD80 oder HLA DR auf der dendritischen Zelle erhöht wird.

3. Verfahren nach Anspruch 1 oder 2, des Weiteren umfassend das Co-Aktivieren der dendritischen Zelle durch einen TLR4-Rezeptor, wobei die Zellen die Zytokin- und Chemokinproduktion oder die Sekretion von IL-10, IL-1b, TNFα, IL-12p40 und Kombinationen aus diesen steigern.

4. Verfahren nach Anspruch 3, wobei zur Co-Aktivierung durch den TLR4-Rezeptor mindestens eines/r der folgenden eingesetzt wird: ein TLR4-Ligand, ein TLR4-Antikörper oder ein Fragment davon, ein Anti-TLR4-anti-Dectin-1-Hybrid-Antikörper oder ein Fragment davon oder ein Anti-TLR4-anti-Dectin-1-Ligandenkonjugat.

5. Verfahren nach einem der Ansprüche 1 bis 4, wobei die erste Hälfte des Cohesin/Dockerin-Paars mit einer zweiten Hälfte eines Cohesin/Dockerin-Paars assoziiert ist, die an ein Antigen oder an ein Zytokin oder an eine Kombination aus einem Zytokin und einem Antigen gebunden ist.

6. Verfahren nach einem der Ansprüche 1 bis 5, des Weiteren umfassend das in Kontakt bringen der dendritischen Zelle mit Monozyten, mononukleären Zellen aus peripherem Blut, B-Zellen oder Kombinationen aus diesen.

7. Impfstoff umfassend eine durch einen Dectin-1-spezifischen Antikörper oder ein Fragment davon aktivierte dendritische Zelle, bevorzugt umfassend mindestens eine der SEQ ID NRs: 1 bis 13, wobei der Anti-Dectin-1-spezifische Antikörper oder das Fragment davon an eine erste Hälfte eines Cohesin/Dockerin-Paars gebunden ist, die mit einer zweiten Hälfte eines Cohesin/Dockerin-Paars assoziiert ist, die an ein Antigen oder an ein Zytokin oder an eine Kombination aus einem Zytokin und einem Antigen gebunden ist.

## Revendications

1. Procédé permettant d'augmenter l'efficacité de la présentation de l'antigène par une cellule dendritique exprimant la dectine 1 comprenant la mise en contact de la cellule dendritique avec un anticorps spécifique anti-dectine 1 ou un fragment de celui-ci, où l'anticorps spécifique anti-dectine 1 ou un fragment de celui-ci est lié à une première moitié d'un couple cohésine/dockerine et où l'anticorps spécifique anti-dectine 1 ou un fragment de celui-ci est capable d'activer la cellule dendritique, où la cellule dendritique est activée.

2. Procédé selon la revendication 1, comprenant en outre l'activation de la cellule dendritique avec du GM-CSF, de l'interféron alpha ou de l'IL-4, ou des combinaisons de ceux-ci, où le contact avec l'anticorps spécifique anti-dectine 1 ou un fragment de celui-ci augmente l'expression à la surface de CD83, CD86, CD80 ou HLA DR sur la cellule dendritique.

3. Procédé selon les revendications 1 ou 2, comprenant en outre la coactivation de la cellule dendritique par l'intermédiaire d'un récepteur TLR4, où les cellules augmentent la production de cytokines et de chimiokines, ou la sécrétion d'IL-10, d'IL-1b, de TNFα, d'IL-12p40 et de combinaisons de ceux-ci.

4. Procédé selon la revendication 3, dans lequel la coactivation par l'intermédiaire du récepteur TLR4 emploie au moins un ligand du TLR4, un anticorps anti-TLR4 ou des fragments de celui-ci, un anticorps hybride anti-TLR4-anti-dectine 1 ou un fragment de celui-ci, un conjugué anti-TLR4-anti-dectine 1-ligand.

5. Procédé selon l'une quelconque des revendications 1 à 4, dans lequel la première moitié du couple cohésine/dockerine est associé à une seconde moitié d'un couple cohésine/dockerine qui est lié à un antigène ou à une cytokine ou à une combinaison de cytokine et d'antigène.

6. Procédé selon l'une quelconque des revendications 1 à 5, comprenant en outre la mise en contact de la cellule dendritique avec des monocytes, des cellules mononucléées du sang périphérique, des cellules B, ou des combinaisons de ceux-ci.

7. Vaccin comprenant une cellule dendritique activée avec un anticorps spécifique anti-dectine 1 ou un fragment de celui-ci, comprenant de préférence au moins l'une de SEQ ID NO : 1 à 13, dans lequel l'anticorps spécifique anti-dectine 1 ou un fragment de celui-ci est lié à une première moitié du couple cohésine/dockerine associé avec une seconde moitié d'un couple cohésine/dockerine qui est liée à un antigène ou à une cytokine ou à une combinaison de cytokine et d'antigène.


### SEQUENCE LISTING

<110> Banchereau, Jacques
   Zurawski, Gerard
   Zurawski, Sandra
   Oh, SangKon
   Ni, Ling
<120> THERAPEUTIC APPLICATIONS OF ACTIVATION OF HUMAN ANTIGEN-PRESENTING CELLS THROUGH DECTIN-1
<130> BHCS:1047
<150> US 60/ 891, 425
   <151> 2007-02-23
<160> 25
<170> PatentIn version 3. 4
<210> 1
   <211> 186
   <212> DNA
   <213> Artificial
<220>
   <223> Chemically Synthesized Oligonucleotide
<400> 1
   gacaccaccg aggcccgcca cccccacccc cccgtgacca cccccaccac caccgaccgg      60
   aagggcacca ccgccgagga gctggccggc atcggcatcc tgaccgtgat cctgggcggc     120
   aagcggacca acaacagcac ccccaccaag ggcgaattct gcagatatcc atcacactgg     180
   cggccg 186
<210> 2
   <211> 61
   <212> PRT
   <213> Artificial
<220>
   <223> Chemically Synthesized Polypeptide
<400> 2
   Asp Thr Thr Glu Ala Arg His Pro His Pro Pro Val Thr Thr Pro Thr
   1 5 10 15
   Thr Asp Arg Lys Gly Thr Thr Ala Glu Glu Leu Ala Gly Ile Gly Ile
   20 25 30
   Leu Thr Val Ile Leu Gly Gly Lys Arg Thr Asn Asn Ser Thr Pro Thr
   35 40 45
   Lys Gly Glu Phe Cys Arg Tyr Pro Ser His Trp Arg Pro
   50 55 60
<210> 3
   <211> 447
   <212> PRT
   <213> Artificial
<220>
   <223> Chemically Synthesized Polypeptide
<400> 3
   Gln Val Gln Leu Lys Glu Ser Gly Pro Gly Leu Val Ala Pro Ser Gln
   1               5                   10                  15
   Ser Leu Ser Ile Thr Cys Ser Val Ser Gly Phe Ser Leu Ser Asn Tyr
               20                  25                  30
   Asp Ile Ser Trp Ile Arg Gln Pro Pro Gly Lys Gly Leu Glu Trp Leu
           35                  40                  45
   Gly Val Met Trp Thr Gly Gly Gly Ala Asn Tyr Asn Ser Ala Phe Met
       50                  55                  60
   Ser Arg Leu Ser Ile Asn Lys Asp Asn Ser Lys Ser Gln Val Phe Leu
   65                  70                  75                  80
   Lys Met Asn Asn Leu Gln Thr Asp Asp Thr Ala Ile Tyr Tyr Cys Val
                   85                  90                  95
   Arg Asp Ala Val Arg Tyr Trp Asn Phe Asp Val Trp Gly Ala Gly Thr
               100                 105                 110
   Thr Val Thr Val Ser Ser Ala Lys Thr Lys Gly Pro Ser Val Phe Pro
           115                 120                 125
   Leu Ala Pro Cys Ser Arg Ser Thr Ser Glu Ser Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Lys Thr Tyr Thr Cys Asn Val Asp His Lys Pro Ser
           195                 200                 205
   Asn Thr Lys Val Asp Lys Arg Val Glu Ser Lys Tyr Gly Pro Pro Cys
       210                 215                 220
   Pro Pro Cys Pro Ala Pro Glu Phe Glu Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser Gln Glu Asp Pro Glu Val Gln
   260 265 270
   Phe Asn Trp Tyr Val Asp Gly Val Glu Val His Asn Ala Lys Thr Lys
   275 280 285
   Pro Arg Glu Glu Gln Phe Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Gly Leu Pro Ser Ser Ile Glu Lys Thr Ile Ser Lys
   325 330 335
   Ala Lys Gly Gln Pro Arg Glu Pro Gln Val Tyr Thr Leu Pro Pro Ser
   340 345 350
   Gln Glu Glu Met Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Arg Leu Thr Val Asp Lys Ser Arg Trp Gln
   405 410 415
   Glu Gly Asn Val Phe Ser Cys Ser Val Met His Glu Ala Leu His Asn
   420 425 430
   His Tyr Thr Gln Lys Ser Leu Ser Leu Ser Leu Gly Lys Ala Ser
   435 440 445
<210> 4
   <211> 213
   <212> PRT
   <213> Artificial
<220>
   <223> Chemically Synthesized Polypeptide
<400> 4
   Gln Ile Val Leu Ser Gln Ser Pro Ala Ile Leu Ser Ala Ser Pro Gly
   1 5 10 15
   Glu Lys Val Thr Met Thr Cys Arg Ala Ser Ser Ser Val Ser Tyr Ile
   20 25 30
   His Trp Tyr Gln Gln Lys Pro Gly Ser Ser Pro Lys Pro Trp Ile Tyr
   35 40 45
   Ala Thr Ser His Leu Ala Ser Gly Val Pro Ala Arg Phe Ser Gly Ser
   50 55 60
   Gly Ser Gly Thr Ser Tyr Ser Leu Thr Ile Ser Arg Val Glu Ala Glu
   65 70 75 80
   Asp Thr Ala Thr Tyr Tyr Cys Gln Gln Trp Ser Ser Asn Pro Phe Thr
   85 90 95
   Phe Gly Ser Gly Thr Lys Leu Glu Ile Lys Arg Thr Val Ala Ala Pro
   100 105 110
   Ser Val Phe Ile Phe Pro Pro Ser Asp Glu Gln Leu Lys Ser Gly Thr
   115 120 125
   Ala Ser Val Val Cys Leu Leu Asn Asn Phe Tyr Pro Arg Glu Ala Lys
   130 135 140
   Val Gln Trp Lys Val Asp Asn Ala Leu Gln Ser Gly Asn Ser Gln Glu
   145 150 155 160
   Ser Val Thr Glu Gln Asp Ser Lys Asp Ser Thr Tyr Ser Leu Ser Ser
   165 170 175
   Thr Leu Thr Leu Ser Lys Ala Asp Tyr Glu Lys His Lys Val Tyr Ala
   180 185 190
   Cys Glu Val Thr His Gln Gly Leu Ser Ser Pro Val Thr Lys Ser Phe
   195 200 205
   Asn Arg Gly Glu Cys
   210
<210> 5
   <211> 450
   <212> PRT
   <213> Artificial
<220>
   <223> Chemically Synthesized Polypeptide
<400> 5
   Gln Val Gln Leu Gln Gln Ser Gly Ala Glu Leu Ala Arg Pro Gly Ala
   1 5 10 15
   Ser Val Lys Met Ser Cys Lys Ala Ser Gly Tyr Thr Phe Thr Thr Tyr
   20 25 30
   Thr Met His Trp Val Lys Gln Arg Pro Gly Gln Gly Leu Glu Trp Ile
   35 40 45
   Gly Tyr Ile Asn Pro Ser Ser Gly Tyr Thr Asn Tyr Asn Gln Lys Phe
   50 55 60
   Lys Asp Lys Ala Thr Leu Thr Ala Asp Lys Ser Ser Ser Thr Ala Ser
   65 70 75 80
   Met Gln Leu Ser Ser Leu Thr Ser Glu Asp Ser Ala Val Tyr Tyr Cys
   85 90 95
   Ala Arg Glu Arg Ala Val Leu Val Pro Tyr Ala Met Asp Tyr Trp Gly
   100 105 110
   Gln Gly Thr Ser Val Thr Val Ser Ser Ala Lys Thr Lys Gly Pro Ser
   115 120 125
   Val Phe Pro Leu Ala Pro Cys Ser Arg Ser Thr Ser Glu Ser Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Lys Thr Tyr Thr Cys Asn Val Asp His
   195 200 205
   Lys Pro Ser Asn Thr Lys Val Asp Lys Arg Val Glu Ser Lys Tyr Gly
   210 215 220
   Pro Pro Cys Pro Pro Cys Pro Ala Pro Glu Phe Glu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser Gln Glu Asp Pro
   260 265 270
   Glu Val Gln Phe Asn Trp Tyr Val Asp Gly Val Glu Val His Asn Ala
   275 280 285
   Lys Thr Lys Pro Arg Glu Glu Gln Phe Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Gly Leu Pro Ser Ser Ile Glu Lys Thr
   325 330 335
   Ile Ser Lys Ala Lys Gly Gln Pro Arg Glu Pro Gln Val Tyr Thr Leu
   340 345 350
   Pro Pro Ser Gln Glu Glu Met Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu Gly Lys
   435 440 445
   Ala Ser
   450
<210> 6
   <211> 213
   <212> PRT
   <213> Artificial
<220>
   <223> Chemically Synthesized Polypeptide
<400> 6
   Gln Ile Val Leu Thr Gln Ser Pro Ala Val Met Ser Ala Ser Pro Gly
   1 5 10 15
   Glu Lys Val Thr Ile Thr Cys Thr Ala Ser Ser Ser Leu Ser Tyr Met
   20 25 30
   His Trp Phe Gln Gln Lys Pro Gly Thr Ser Pro Lys Leu Trp Leu Tyr
   35 40 45
   Ser Thr Ser Ile Leu Ala Ser Gly Val Pro Thr Arg Phe Ser Gly Ser
   50 55 60
   Gly Ser Gly Thr Ser Tyr Ser Leu Thr Ile Ser Arg Met Glu Ala Glu
   65 70 75 80
   Asp Ala Ala Thr Tyr Tyr Cys Gln Gln Arg Ser Ser Ser Pro Phe Thr
   85 90 95
   Phe Gly Ser Gly Thr Lys Leu Glu Ile Lys Arg Thr Val Ala Ala Pro
   100 105 110
   Ser Val Phe Ile Phe Pro Pro Ser Asp Glu Gln Leu Lys Ser Gly Thr
   115 120 125
   Ala Ser Val Val Cys Leu Leu Asn Asn Phe Tyr Pro Arg Glu Ala Lys
   130 135 140
   Val Gln Trp Lys Val Asp Asn Ala Leu Gln Ser Gly Asn Ser Gln Glu
   145 150 155 160
   Ser Val Thr Glu Gln Asp Ser Lys Asp Ser Thr Tyr Ser Leu Ser Ser
   165 170 175
   Thr Leu Thr Leu Ser Lys Ala Asp Tyr Glu Lys His Lys Val Tyr Ala
   180 185 190
   Cys Glu Val Thr His Gln Gly Leu Ser Ser Pro Val Thr Lys Ser Phe
   195 200 205
   Asn Arg Gly Glu Cys
   210
<210> 7
   <211> 448
   <212> PRT
   <213> Artificial
<220>
   <223> Chemically Synthesized Polypeptide
<400> 7
   Glu Val Gln Leu Gln Gln Ser Gly Pro Glu Leu Glu Lys Pro Gly Ala
   1 5 10 15
   Ser Val Lys Ile Ser Cys Lys Ala Ser Gly Tyr Ser Phe Thr Gly Tyr
   20 25 30
   Asn Met Asn Trp Val Lys Gln Ser Asn Gly Lys Ser Leu Glu Trp Ile
   35 40 45
   Gly Asn Ile Asp Pro Tyr Tyr Gly Asp Thr Asn Tyr Asn Gln Lys Phe
   50 55 60
   Lys Gly Lys Ala Thr Leu Thr Val Asp Lys Ser Ser Ser Thr Ala Tyr
   65 70 75 80
   Met His Leu Lys Ser Leu Thr Ser Glu Asp Ser Ala Val Tyr Tyr Cys
   85 90 95
   Ala Arg Pro Tyr Gly Ser Glu Ala Tyr Phe Ala Tyr Trp Gly Gln Gly
   100 105 110
   Thr Leu Val Thr Val Ser Ala Ala Lys Thr Lys Gly Pro Ser Val Phe
   115 120 125
   Pro Leu Ala Pro Cys Ser Arg Ser Thr Ser Glu Ser Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Lys Thr Tyr Thr Cys Asn Val Asp His Lys Pro
   195 200 205
   Ser Asn Thr Lys Val Asp Lys Arg Val Glu Ser Lys Tyr Gly Pro Pro
   210 215 220
   Cys Pro Pro Cys Pro Ala Pro Glu Phe Glu Gly Gly Pro Ser Val Phe
   225 230 235 240
   Leu Phe Pro Pro Lys Pro Lys Asp Thr Leu Met Ile Ser Arg Thr Pro
   245 250 255
   Glu Val Thr Cys Val Val Val Asp Val Ser Gln Glu Asp Pro Glu Val
   260 265 270
   Gln Phe Asn Trp Tyr Val Asp Gly Val Glu Val His Asn Ala Lys Thr
   275 280 285
   Lys Pro Arg Glu Glu Gln Phe Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Gly Leu Pro Ser Ser Ile Glu Lys Thr Ile Ser
   325 330 335
   Lys Ala Lys Gly Gln Pro Arg Glu Pro Gln Val Tyr Thr Leu Pro Pro
   340 345 350
   Ser Gln Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Arg Leu Thr Val Asp Lys Ser Arg Trp
   405 410 415
   Gln Glu Gly Asn Val Phe Ser Cys Ser Val Met His Glu Ala Leu His
   420 425 430
   Asn His Tyr Thr Gln Lys Ser Leu Ser Leu Ser Leu Gly Lys Ala Ser
   435 440 445
<210> 8
   <211> 214
   <212> PRT
   <213> Artificial
<220>
   <223> Chemically Synthesized Polypeptide
<400> 8
   Asp Ile Val Met Thr Gln Ser Pro Ala Thr Leu Ser Val Thr Pro Gly
   1 5 10 15
   Asp Arg Val Ser Leu Ser Cys Arg Ala Ser Gln Ser Ile Ser Asp Tyr
   20 25 30
   Leu His Trp Tyr Gln Gln Lys Ser His Glu Ser Pro Arg Leu Leu Ile
   35 40 45
   Lys Tyr Ala Ala Gln Ser Ile Ser Gly Ile Pro Ser Arg Phe Ser Gly
   50 55 60
   Ser Gly Ser Gly Ser Asp Phe Thr Leu Ser Ile Asn Gly Val Glu Pro
   65 70 75 80
   Glu Asp Val Gly Val Tyr Tyr Cys Gln Asn Gly His Ser Phe Pro Tyr
   85 90 95
   Thr Phe Gly Gly Gly Thr Lys Leu Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 9
   <211> 781
   <212> PRT
   <213> Artificial
<220>
   <223> Chemically Synthesized Polypeptide
<400> 9
   Gln Val Gln Leu Lys Glu Ser Gly Pro Gly Leu Val Ala Pro Ser Gln
   1 5 10 15
   Ser Leu Ser Ile Thr Cys Ser Val Ser Gly Phe Ser Leu Ser Asn Tyr
   20 25 30
   Asp Ile Ser Trp Ile Arg Gln Pro Pro Gly Lys Gly Leu Glu Trp Leu
   35 40 45
   Gly Val Met Trp Thr Gly Gly Gly Ala Asn Tyr Asn Ser Ala Phe Met
   50 55 60
   Ser Arg Leu Ser Ile Asn Lys Asp Asn Ser Lys Ser Gln Val Phe Leu
   65 70 75 80
   Lys Met Asn Asn Leu Gln Thr Asp Asp Thr Ala Ile Tyr Tyr Cys Val
   85 90 95
   Arg Asp Ala Val Arg Tyr Trp Asn Phe Asp Val Trp Gly Ala Gly Thr
   100 105 110
   Thr Val Thr Val Ser Ser Ala Lys Thr Lys Gly Pro Ser Val Phe Pro
   115 120 125
   Leu Ala Pro Cys Ser Arg Ser Thr Ser Glu Ser Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Lys Thr Tyr Thr Cys Asn Val Asp His Lys Pro Ser
   195 200 205
   Asn Thr Lys Val Asp Lys Arg Val Glu Ser Lys Tyr Gly Pro Pro Cys
   210 215 220
   Pro Pro Cys Pro Ala Pro Glu Phe Glu Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser Gln Glu Asp Pro Glu Val Gln
   260 265 270
   Phe Asn Trp Tyr Val Asp Gly Val Glu Val His Asn Ala Lys Thr Lys
   275 280 285
   Pro Arg Glu Glu Gln Phe Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Gly Leu Pro Ser Ser Ile Glu Lys Thr Ile Ser Lys
   325 330 335
   Ala Lys Gly Gln Pro Arg Glu Pro Gln Val Tyr Thr Leu Pro Pro Ser
   340 345 350
   Gln Glu Glu Met Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Arg Leu Thr Val Asp Lys Ser Arg Trp Gln
   405 410 415
   Glu Gly Asn Val Phe Ser Cys Ser Val Met His Glu Ala Leu His Asn
   420 425 430
   His Tyr Thr Gln Lys Ser Leu Ser Leu Ser Leu Gly Lys Ala Ser Asp
   435 440 445
   Thr Thr Glu Pro Ala Thr Pro Thr Thr Pro Val Thr Thr Asp Thr Ile
   450 455 460
   Cys Ile Gly Tyr His Ala Asn Asn Ser Thr Asp Thr Val Asp Thr Val
   465 470 475 480
   Leu Glu Lys Asn Val Thr Val Thr His Ser Val Asn Leu Leu Glu Asp
   485 490 495
   Ser His Asn Gly Lys Leu Cys Arg Leu Lys Gly Ile Ala Pro Leu Gln
   500 505 510
   Leu Gly Lys Cys Asn Ile Ala Gly Trp Leu Leu Gly Asn Pro Glu Cys
   515 520 525
   Asp Pro Leu Leu Pro Val Arg Ser Trp Ser Tyr Ile Val Glu Thr Pro
   530 535 540
   Asn Ser Glu Asn Gly Ile Cys Tyr Pro Gly Asp Phe Ile Asp Tyr Glu
   545 550 555 560
   Glu Leu Arg Glu Gln Leu Ser Ser Val Ser Ser Phe Glu Arg Phe Glu
   565 570 575
   Ile Phe Pro Lys Glu Ser Ser Trp Pro Asn His Asn Thr Asn Gly Val
   580 585 590
   Thr Ala Ala Cys Ser His Glu Gly Lys Ser Ser Phe Tyr Arg Asn Leu
   595 600 605
   Leu Trp Leu Thr Glu Lys Glu Gly Ser Tyr Pro Lys Leu Lys Asn Ser
   610 615 620
   Tyr Val Asn Lys Lys Gly Lys Glu Val Leu Val Leu Trp Gly Ile His
   625 630 635 640
   His Pro Pro Asn Ser Lys Glu Gln Gln Asn Leu Tyr Gln Asn Glu Asn
   645 650 655
   Ala Tyr Val Ser Val Val Thr Ser Asn Tyr Asn Arg Arg Phe Thr Pro
   660 665 670
   Glu Ile Ala Glu Arg Pro Lys Val Arg Asp Gln Ala Gly Arg Met Asn
   675 680 685
   Tyr Tyr Trp Thr Leu Leu Lys Pro Gly Asp Thr Ile Ile Phe Glu Ala
   690 695 700
   Asn Gly Asn Leu Ile Ala Pro Met Tyr Ala Phe Ala Leu Ser Arg Gly
   705 710 715 720
   Phe Gly Ser Gly Ile Ile Thr Ser Asn Ala Ser Met His Glu Cys Asn
   725 730 735
   Thr Lys Cys Gln Thr Pro Leu Gly Ala Ile Asn Ser Ser Leu Pro Tyr
   740 745 750
   Gln Asn Ile His Pro Val Thr Ile Gly Glu Cys Leu Lys Tyr Val Arg
   755 760 765
   Ser Ala Lys Leu Arg Met Val His His His His His His
   770 775 780
<210> 10
   <211> 781
   <212> PRT
   <213> Artificial
<220>
   <223> Chemically Synthesized Polypeptide
<400> 10
   Gln Val Gln Leu Lys Glu Ser Gly Pro Gly Leu Val Ala Pro Ser Gln
   1 5 10 15
   Ser Leu Ser Ile Thr Cys Ser Val Ser Gly Phe Ser Leu Ser Asn Tyr
   20 25 30
   Asp Ile Ser Trp Ile Arg Gln Pro Pro Gly Lys Gly Leu Glu Trp Leu
   35 40 45
   Gly Val Met Trp Thr Gly Gly Gly Ala Asn Tyr Asn Ser Ala Phe Met
   50 55 60
   Ser Arg Leu Ser Ile Asn Lys Asp Asn Ser Lys Ser Gln Val Phe Leu
   65 70 75 80
   Lys Met Asn Asn Leu Gln Thr Asp Asp Thr Ala Ile Tyr Tyr Cys Val
   85 90 95
   Arg Asp Ala Val Arg Tyr Trp Asn Phe Asp Val Trp Gly Ala Gly Thr
   100 105 110
   Thr Val Thr Val Ser Ser Ala Lys Thr Lys Gly Pro Ser Val Phe Pro
   115 120 125
   Leu Ala Pro Cys Ser Arg Ser Thr Ser Glu Ser Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Lys Thr Tyr Thr Cys Asn Val Asp His Lys Pro Ser
   195 200 205
   Asn Thr Lys Val Asp Lys Arg Val Glu Ser Lys Tyr Gly Pro Pro Cys
   210 215 220
   Pro Pro Cys Pro Ala Pro Glu Phe Glu Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser Gln Glu Asp Pro Glu Val Gln
   260 265 270
   Phe Asn Trp Tyr Val Asp Gly Val Glu Val His Asn Ala Lys Thr Lys
   275 280 285
   Pro Arg Glu Glu Gln Phe Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Gly Leu Pro Ser Ser Ile Glu Lys Thr Ile Ser Lys
   325 330 335
   Ala Lys Gly Gln Pro Arg Glu Pro Gln Val Tyr Thr Leu Pro Pro Ser
   340 345 350
   Gln Glu Glu Met Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Arg Leu Thr Val Asp Lys Ser Arg Trp Gln
   405 410 415
   Glu Gly Asn Val Phe Ser Cys Ser Val Met His Glu Ala Leu His Asn
   420 425 430
   His Tyr Thr Gln Lys Ser Leu Ser Leu Ser Leu Gly Lys Ala Ser Asp
   435 440 445
   Thr Thr Glu Pro Ala Thr Pro Thr Thr Pro Val Thr Thr Asp Gln Ile
   450 455 460
   Cys Ile Gly Tyr His Ala Asn Asn Ser Thr Glu Gln Val Asp Thr Ile
   465 470 475 480
   Met Glu Lys Asn Val Thr Val Thr His Ala Gln Asp Ile Leu Glu Lys
                   485                 490                 495
   Lys His Asn Gly Lys Leu Cys Asp Leu Asp Gly Val Lys Pro Leu Ile
               500                 505                 510
   Leu Arg Asp Cys Ser Val Ala Gly Trp Leu Leu Gly Asn Pro Met Cys
           515                 520                 525
   Asp Glu Phe Ile Asn Val Pro Glu Trp Ser Tyr Ile Val Glu Lys Ala
       530                 535                 540
   Asn Pro Val Asn Asp Leu Cys Tyr Pro Gly Asp Phe Asn Asp Tyr Glu
   545                 550                 555                 560
   Glu Leu Lys His Leu Leu Ser Arg Ile Asn His Phe Glu Lys Ile Gln
                   565                 570                 575
   Ile Ile Pro Lys Ser Ser Trp Ser Ser His Glu Ala Ser Leu Gly Val
               580                 585                 590
   Ser Ser Ala Cys Pro Tyr Gln Gly Lys Ser Ser Phe Phe Arg Asn Val
           595                 600                 605
   Val Trp Leu Ile Lys Lys Asn Ser Thr Tyr Pro Thr Ile Lys Arg Ser
       610                 615                 620
   Tyr Asn Asn Thr Asn Gln Glu Asp Leu Leu Val Leu Trp Gly Ile His
   625                 630                 635                 640
   His Pro Asn Asp Ala Ala Glu Gln Thr Lys Leu Tyr Gln Asn Pro Thr
                   645                 650                 655
   Thr Tyr Ile Ser Val Gly Thr Ser Thr Leu Asn Gln Arg Leu Val Pro
               660                 665                 670
   Arg Ile Ala Thr Arg Ser Lys Val Asn Gly Gln Ser Gly Arg Met Glu
           675                 680                 685
   Phe Phe Trp Thr Ile Leu Lys Pro Asn Asp Ala Ile Asn Phe Glu Ser
       690                 695                 700
   Asn Gly Asn Phe Ile Ala Pro Glu Tyr Ala Tyr Lys Ile Val Lys Lys
   705                 710                 715                 720
   Gly Asp Ser Thr Ile Met Lys Ser Glu Leu Glu Tyr Gly Asn Cys Asn
                   725                 730                 735
   Thr Lys Cys Gln Thr Pro Met Gly Ala Ile Asn Ser Ser Met Pro Phe
               740                 745                 750
   His Asn Ile His Pro Leu Thr Ile Gly Glu Cys Pro Lys Tyr Val Lys
   755 760 765
   Ser Asn Arg Leu Val Leu Ala His His His His His His
   770 775 780
<210> 11
   <211> 695
   <212> PRT
   <213> Artificial
<220>
   <223> Chemically Synthesized Polypeptide
<400> 11
   Glu Val Gln Leu Gln Gln Ser Gly Pro Glu Leu Glu Lys Pro Gly Ala
   1               5                   10                  15
   Ser Val Lys Ile Ser Cys Lys Ala Ser Gly Tyr Ser Phe Thr Gly Tyr
               20                  25                  30
   Asn Met Asn Trp Val Lys Gln Ser Asn Gly Lys Ser Leu Glu Trp Ile
           35                  40                  45
   Gly Asn Ile Asp Pro Tyr Tyr Gly Asp Thr Asn Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met His Leu Lys Ser Leu Thr Ser Glu Asp Ser Ala Val Tyr Tyr Cys
                   85                  90                  95
   Ala Arg Pro Tyr Gly Ser Glu Ala Tyr Phe Ala Tyr Trp Gly Gln Gly
               100                 105                 110
   Thr Leu Val Thr Val Ser Ala Ala Lys Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Lys Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Glu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Phe Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Arg Leu Thr Val Asp Lys Ser Arg Trp
                   405                 410                 415
   Gln Glu Gly Asn Val Phe Ser Cys Ser Val Met His Glu Ala Leu His
               420                 425                 430
   Asn His Tyr Thr Gln Lys Ser Leu Ser Leu Ser Leu Gly Lys Ala Ser
           435                 440                 445
   Asp Met Ala Lys Lys Glu Thr Val Trp Arg Leu Glu Glu Phe Gly Arg
       450                 455                 460
   Pro Ile Val Gln Asn Ile Gln Gly Gln Met Val His Gln Ala Ile Ser
   465                 470                 475                 480
   Pro Arg Thr Leu Asn Ala Trp Val Lys Val Val Glu Glu Lys Ala Phe
                   485                 490                 495
   Ser Pro Glu Val Ile Pro Met Phe Ser Ala Leu Ser Glu Gly Ala Thr
               500                 505                 510
   Pro Gln Asp Leu Asn Thr Met Leu Asn Thr Val Gly Gly His Gln Ala
           515                 520                 525
   Ala Met Gln Met Leu Lys Glu Thr Ile Asn Glu Glu Ala Ala Glu Trp
       530                 535                 540
   Asp Arg Val His Pro Val His Ala Gly Pro Ile Ala Pro Gly Gln Met
   545                 550                 555                 560
   Arg Glu Pro Arg Gly Ser Asp Ile Ala Gly Thr Thr Ser Thr Leu Gln
                   565                 570                 575
   Glu Gln Ile Gly Trp Met Thr Asn Asn Pro Pro Ile Pro Val Gly Glu
               580                 585                 590
   Ile Tyr Lys Arg Trp Ile Ile Leu Gly Leu Asn Lys Ile Val Arg Met
           595                 600                 605
   Tyr Ser Pro Thr Ser Ile Leu Asp Ile Arg Gln Gly Pro Lys Glu Pro
       610                 615                 620
   Phe Arg Asp Tyr Val Asp Arg Phe Tyr Lys Thr Leu Arg Ala Glu Gln
   625                 630                 635                 640
   Ala Ser Gln Glu Val Lys Asn Trp Met Thr Glu Thr Leu Leu Val Gln
                   645                 650                 655
   Asn Ala Asn Pro Asp Cys Lys Thr Ile Leu Lys Ala Leu Gly Pro Ala
               660                 665                 670
   Ala Thr Leu Glu Glu Met Met Thr Ala Cys Gln Gly Val Gly Gly Pro
           675                 680                 685
   Gly His Lys Ala Arg Val Leu
       690                 695
<210> 12
   <211> 712
   <212> PRT
   <213> Art i f i ci al
<220>
   <223> Chemicaly Synthesized Polypeptide
<400> 12
   Glu Val Gln Leu Gln Gln Ser Gly Pro Glu Leu Glu Lys Pro Gly Ala
   1               5                   10                  15
   Ser Val Lys Ile Ser Cys Lys Ala Ser Gly Tyr Ser Phe Thr Gly Tyr
   20 25 30
   Asn Met Asn Trp Val Lys Gln Ser Asn Gly Lys Ser Leu Glu Trp Ile
   35 40 45
   Gly Asn Ile Asp Pro Tyr Tyr Gly Asp Thr Asn Tyr Asn Gln Lys Phe
   50 55 60
   Lys Gly Lys Ala Thr Leu Thr Val Asp Lys Ser Ser Ser Thr Ala Tyr
   65 70 75 80
   Met His Leu Lys Ser Leu Thr Ser Glu Asp Ser Ala Val Tyr Tyr Cys
   85 90 95
   Ala Arg Pro Tyr Gly Ser Glu Ala Tyr Phe Ala Tyr Trp Gly Gln Gly
   100 105 110
   Thr Leu Val Thr Val Ser Ala Ala Lys Thr Lys Gly Pro Ser Val Phe
   115 120 125
   Pro Leu Ala Pro Cys Ser Arg Ser Thr Ser Glu Ser Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Lys Thr Tyr Thr Cys Asn Val Asp His Lys Pro
   195 200 205
   Ser Asn Thr Lys Val Asp Lys Arg Val Glu Ser Lys Tyr Gly Pro Pro
   210 215 220
   Cys Pro Pro Cys Pro Ala Pro Glu Phe Glu Gly Gly Pro Ser Val Phe
   225 230 235 240
   Leu Phe Pro Pro Lys Pro Lys Asp Thr Leu Met Ile Ser Arg Thr Pro
   245 250 255
   Glu Val Thr Cys Val Val Val Asp Val Ser Gln Glu Asp Pro Glu Val
   260 265 270
   Gln Phe Asn Trp Tyr Val Asp Gly Val Glu Val His Asn Ala Lys Thr
   275 280 285
   Lys Pro Arg Glu Glu Gln Phe Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Gly Leu Pro Ser Ser Ile Glu Lys Thr Ile Ser
   325 330 335
   Lys Ala Lys Gly Gln Pro Arg Glu Pro Gln Val Tyr Thr Leu Pro Pro
   340 345 350
   Ser Gln Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Arg Leu Thr Val Asp Lys Ser Arg Trp
   405 410 415
   Gln Glu Gly Asn Val Phe Ser Cys Ser Val Met His Glu Ala Leu His
   420 425 430
   Asn His Tyr Thr Gln Lys Ser Leu Ser Leu Ser Leu Gly Lys Ala Ser
   435 440 445
   Asp Thr Thr Glu Pro Ala Thr Pro Thr Thr Pro Val Thr Thr Pro Thr
   450 455 460
   Thr Thr Leu Leu Ala Pro Leu Ile Leu Ser Arg Ile Val Gly Gly Trp
   465 470 475 480
   Glu Cys Glu Lys His Ser Gln Pro Trp Gln Val Leu Val Ala Ser Arg
   485 490 495
   Gly Arg Ala Val Cys Gly Gly Val Leu Val His Pro Gln Trp Val Leu
   500 505 510
   Thr Ala Ala His Cys Ile Arg Asn Lys Ser Val Ile Leu Leu Gly Arg
   515 520 525
   His Ser Leu Phe His Pro Glu Asp Thr Gly Gln Val Phe Gln Val Ser
   530 535 540
   His Ser Phe Pro His Pro Leu Tyr Asp Met Ser Leu Leu Lys Asn Arg
   545 550 555 560
   Phe Leu Arg Pro Gly Asp Asp Ser Ser His Asp Leu Met Leu Leu Arg
   565 570 575
   Leu Ser Glu Pro Ala Glu Leu Thr Asp Ala Val Lys Val Met Asp Leu
   580 585 590
   Pro Thr Gln Glu Pro Ala Leu Gly Thr Thr Cys Tyr Ala Ser Gly Trp
   595 600 605
   Gly Ser Ile Glu Pro Glu Glu Phe Leu Thr Pro Lys Lys Leu Gln Cys
   610 615 620
   Val Asp Leu His Val Ile Ser Asn Asp Val Cys Ala Gln Val His Pro
   625 630 635 640
   Gln Lys Val Thr Lys Phe Met Leu Cys Ala Gly Arg Trp Thr Gly Gly
   645 650 655
   Lys Ser Thr Cys Ser Gly Asp Ser Gly Gly Pro Leu Val Cys Asn Gly
   660 665 670
   Val Leu Gln Gly Ile Thr Ser Trp Gly Ser Glu Pro Cys Ala Leu Pro
   675 680 685
   Glu Arg Pro Ser Leu Tyr Thr Lys Val Val His Tyr Arg Lys Trp Ile
   690 695 700
   Lys Asp Thr Ile Val Ala Asn Pro
   705 710
<210> 13
   <211> 522
   <212> PRT
   <213> Artificial
<220>
   <223> Chemically Synthesized Polypeptide
<400> 13
   Gln Val Gln Leu Lys Glu Ser Gly Pro Gly Leu Val Ala Pro Ser Gln
   1 5 10 15
   Ser Leu Ser Ile Thr Cys Ser Val Ser Gly Phe Ser Leu Ser Asn Tyr
   20 25 30
   Asp Ile Ser Trp Ile Arg Gln Pro Pro Gly Lys Gly Leu Glu Trp Leu
   35 40 45
   Gly Val Met Trp Thr Gly Gly Gly Ala Asn Tyr Asn Ser Ala Phe Met
   50 55 60
   Ser Arg Leu Ser Ile Asn Lys Asp Asn Ser Lys Ser Gln Val Phe Leu
   65 70 75 80
   Lys Met Asn Asn Leu Gln Thr Asp Asp Thr Ala Ile Tyr Tyr Cys Val
   85 90 95
   Arg Asp Ala Val Arg Tyr Trp Asn Phe Asp Val Trp Gly Ala Gly Thr
   100 105 110
   Thr Val Thr Val Ser Ser Ala Lys Thr Lys Gly Pro Ser Val Phe Pro
   115 120 125
   Leu Ala Pro Cys Ser Arg Ser Thr Ser Glu Ser Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Lys Thr Tyr Thr Cys Asn Val Asp His Lys Pro Ser
   195 200 205
   Asn Thr Lys Val Asp Lys Arg Val Glu Ser Lys Tyr Gly Pro Pro Cys
   210 215 220
   Pro Pro Cys Pro Ala Pro Glu Phe Glu Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser Gln Glu Asp Pro Glu Val Gln
   260 265 270
   Phe Asn Trp Tyr Val Asp Gly Val Glu Val His Asn Ala Lys Thr Lys
   275 280 285
   Pro Arg Glu Glu Gln Phe Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Gly Leu Pro Ser Ser Ile Glu Lys Thr Ile Ser Lys
   325 330 335
   Ala Lys Gly Gln Pro Arg Glu Pro Gln Val Tyr Thr Leu Pro Pro Ser
   340 345 350
   Gln Glu Glu Met Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Arg Leu Thr Val Asp Lys Ser Arg Trp Gln
   405 410 415
   Glu Gly Asn Val Phe Ser Cys Ser Val Met His Glu Ala Leu His Asn
   420 425 430
   His Tyr Thr Gln Lys Ser Leu Ser Leu Ser Leu Gly Lys Ala Ser Asn
   435 440 445
   Ser Pro Gln Asn Glu Val Leu Tyr Gly Asp Val Asn Asp Asp Gly Lys
   450 455 460
   Val Asn Ser Thr Asp Leu Thr Leu Leu Lys Arg Tyr Val Leu Lys Ala
   465 470 475 480
   Val Ser Thr Leu Pro Ser Ser Lys Ala Glu Lys Asn Ala Asp Val Asn
   485 490 495
   Arg Asp Gly Arg Val Asn Ser Ser Asp Val Thr Ile Leu Ser Arg Tyr
   500 505 510
   Leu Ile Arg Val Ile Glu Lys Leu Pro Ile
   515 520
<210> 14
   <211> 36
   <212> DNA
   <213> Artificial
<220>
   <223> Chemically Synthesized Oligonucleotide
<400> 14
   ggat ggt ggg aagat ggat a cagt t ggt gc agcat c 36
<210> 15
   <211> 48
   <212> DNA
   <213> Art i f i ci al
<220>
   <223> Chemically Synthesized Oligonucleotide
<400> 15
   ct aggaacag t cagcacggg acaaact ct t ct ccacagt g t gacct t c 48
<210> 16
   <211> 37
   <212> DNA
   <213> Artificial
<220>
   <223> Chemically Synthesized Oligonucleotide
<400> 16
   gt cact ggct cagggaaat a gccct t gacc aggcat c 37
<210> 17
   <211> 31
   <212> DNA
   <213> Artificial
<220>
   <223> Chemically Synthesized Oligonucleot ide
<400> 17
   ccaggcat cc t agagt cacc gaggagccag t 31
<210> 18
   <211> 38
   <212> DNA
   <213> Artificial
<220>
   <223> Chemically Synthesized Oligonucleotide
<400> 18
   ggt gct ggag gggacagt ca ct gagct gct cat agt gt 38
<210> 19
   <211> 107
   <212> DNA
   <213> Artificial
<220>
   <223> Chemically Synthesized Oligonucleotide
<400> 19
   ctagttgctg gctaatggac cccaaaggct ccctttcctg gagaatactt ctgtttctct       60
   ccct ggct t t t gagt t gt cg t acggat t aa t t aagggccc act cgag 107
<210> 20
   <211> 17
   <212> DNA
   <213> Artificial
<220>
   <223> Chemically Synthesized Oligonucleotide
<400> 20
   gct agct gat t aat t aa 17
<210> 21
   <211> 100
   <212> DNA
   <213> Artificial
<220>
   <223> Chemically Synthesized Oligonucleotide
<400> 21
   ctagttgctg gctaatggac cccaaaggct ccctttcctg gagaatactt ctgtttctct     60
   ccct ggct t t t gagt t gt cg t acggat t aa t t aagggccc 100
<210> 22
   <211> 46
   <212> DNA
   <213> Artificial
<220>
   <223> Chemically Synthesized Oligonucleotide
<400> 22
   gct agcgat a caacagaacc t gcaacacct acaacacct g taacaa 46
<210> 23
   <211> 29
   <212> DNA
   <213> Artificial
<220>
   <223> Chemically Synthesized Oligonucleotide
<400> 23
   caccatcacc at caccat t g agcggccgc 29
<210> 24
   <211> 70
   <212> DNA
   <213> Artificial
<220>
   <223> Chemically Synthesized Oligonucleotide
<400> 24
   gctagcgata caacagaacc tgcaacacct acaacacctg taacaacacc gacaacaaca     60
   ct t ct agcgc 70
<210> 25
   <211> 119
   <212> DNA
   <213> Artificial
<220>
   <223> Chemically Synthesized Oligonucleotide
<400> 25
   gctagcccca ttctgagccc cctgaccaaa ggcattctgg gctttgtgtt taccctgacc 60
   gtgcccagcg aacgcaaggg tatacttgga ttcgttttca cacttactta agcggccgc 119